(19) Europäisches Patentamt
European Patent Office
Office européen des brevets

(11) Publication number: **0 219 154 B1**

(12) # EUROPEAN PATENT SPECIFICATION

(45) Date of publication of patent specification: **13.11.91**

(51) Int. Cl.5: **A01N 43/08**, C07D 307/24, C07D 307/30, C07D 307/87, C07D 307/92, C07D 307/77, C07D 307/93

(21) Application number: **86201625.0**

(22) Date of filing: **19.09.86**

(54) **Furyl compounds, their use as fungicides and their preparation.**

(30) Priority: **07.10.85 GB 8524659**

(43) Date of publication of application:
**22.04.87 Bulletin 87/17**

(45) Publication of the grant of the patent:
**13.11.91 Bulletin 91/46**

(84) Designated Contracting States:
**AT BE CH DE FR GB IT LI LU NL SE**

(56) References cited:
**DE-A- 2 437 167**
**US-A- 3 235 565**
**US-A- 3 317 567**

**JOURNAL OF THE AMERICAN CHEMICAL SOCIETY, vol. 87, no. 16, 20th August 1965, pages 3657-3665, American Chemical Society, Washington, D.C., US; W.J. LINN et al.: "Tetracyanoethylene oxide. II. Addition to olefins, acetylenes, and aromatics"**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Harkin, Shaun Anthony**
**61 Sandford Road**
**Sittingbourne Kent(GB)**
Inventor: **Slight, Peter John**
**44 Vectis Drive**
**Sittingbourne Kent(GB)**
Inventor: **Hudec, John**
**21 Redhill Close**
**Bassett Southampton Hampshire(GB)**

(74) Representative: **Bennett, David Arthur Horder et al**
**4, York Road**
**London SE1 7NA(GB)**

**EP 0 219 154 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to the use of certain furyl compounds as fungicides, to certain novel compounds per se, and to the preparation of novel compounds according to the invention.

The compounds with which the present invention is concerned may be regarded as 2,2,5,5-tetracyanotetra- and di-hydrofurans. Certain compounds have bivalent groups extending betwen the 3-and 4- positions on the furan ring and are named as 2,2,4,4-tetracyano-3-oxabicyclo compounds.

Some such compounds are known, from the following sources:

J.A.C.S., 1963, 85, 2032-3: this discloses in general terms that tetracyanoethylene oxide adds to olefinic, acetylenic and aromatic compounds to yield products derived from carbon-carbon cleavage of the epoxide molecule. Compounds specifically disclosed are 2,2,5,5,tetracyanotetra- and di-hydrofurans, 1,1,3,3-tetracyano-1,3,3a,7a-tetrahydroisobenzofuran, and its saturated analogue. No use is proposed for the compounds.

Proc. Chem. Soc., 1964, 185-6: this discloses a number of compounds formed by the addition of a dipolarophile to tetracyanoethylene oxide. The dipolarophiles are but-1-ene, cyclopentene, cyclopentadiene, indene, furan, thiophene, cycloheptatriene, cyclo-octatetraene, bicyclo[2,2,1]heptene, bicyclo[2,2,1]-heptadiene, bicyclo [2,2,2]octadiene, benzene, durene, naphthalene, anthracene and phenanthrene. No use is proposed for the resulting adducts.

J.A.C.S., 1965, 87, 3657-65: various adducts of tetracyanoethylene oxide with olefins, acetylenes and aromatics are disclosed. The olefinic adducts are substituted at the 3-or 4- positions on the resulting tetrahydrofuran ring by various substituents including various alkyl groups, phenyl or benzyl groups, chlorine atoms and cyano groups. The acetylenic adducts are substituted at the 3- and 4- positions on the resulting dihydrofuran ring by methyl, phenyl, methoxycarbonyl and propynyl groups. Bicyclic adducts are described, resulting from the addition of tetracyanoethylene oxide to benzene, p-xylene, toluene, cyclohexene, naphthalene, 1,4-dibromonaphthalene, furan, thiophene and 2-chlorothiophene. No use is mentioned for the adducts.

US patent no. 3235565: this discloses a broad class of polycyano-2,5-dihydrofurans, as intermediates to corresponding carboxylic oxides, salts, esters or amides, which may yield condensation polymers. 2,2,5,5-tetracyano-2,5-dihydrofuran compounds specifically disclosed have the following moieties at the 3- and 4- positions: H,H; H,methyl; methyl, methyl; H,phenyl; phenyl, phenyl; methoxycarbonyl, methoxycarbonyl; prop-2-ynyl, methyl; H,3-(2,2,5,5-tetracyano-dihydrofuryl) propyl; H, pent-4-ynyl; n-hexyl, ethoxycarbonyl; methyl, butyl propanoate; octyl, methyl ocsoate; H, benzoyloxymethyl; H,benzyloxymethyl; H, n-dodecyl; ethyl, n-hexyl; H,isopropyl; H,cyclohexyl; H, 3-naphthyl; H, benzyloxycarbonyl; H, 4-chlorophenyl; H, 2-chloromethylphenyl.

US patent no. 3317567: this discloses a broad class of polycyano-2,2,5,5-tetrahydrofurans, as intermediates in the preparation of polymeric materials. 2,2,5,5-tetracyanotetrahydrofuran compounds specifically disclosed have the following moieties at the 3 and 4- positions: H, H; H, acetoxy; methyl, methyl; H, methyl; phenyl, phenyl; H, phenyl; H, bromomethyl; H, acetoxymethyl; H, ethoxymethyl; phenyl, ethoxycarbonyl; H, n-butyl; H, n-octyl; methyl, n-octyl; H, n-hexadecyl; H, but-3-enyl; H, (2,2,5,5-tetracyanotetrahydrofuryl)methyl; H, benzyloxycarbonyl; H, cyanomethyl; H, omega-cyanooctyl; cyanomethyl, cyanomethyl; H, (cyclohexyl)oxycarbonylmethyl; H, (benzyloxycarbonyl)n-hexyl; n-octyl, (methoxycarbonyl)heptyl; H, cyclopentyloxymethyl; H, hexadecyloxymethyl; methyl, (heptylcarbonyloxy)-methyl; H,(pentadecylcarbonyloxy)methyl. Other compounds which may be regarded as 2,2,5,5-tetracyanotetrahydrofuran derivatives are disclosed under the following names:

4,7-dimethyl-1,1,3,3-tetracyano-1,3,3a,7a-tetrahydroisobenzofuran; 1,1,3,3-tetracyano-1,3,3a,7a-tetrahydroisobenzofuran; 4,4,6,6-tetracyano-3a,4,6,6a-tetrahydrothieno[2,3-c]-furan; 3,3-dimethyl-2,2,5,5-tetracyanotetrahydrofuran; 1,1,3,3-tetra- cyano-1,3,3a,9b-tetrahydronaphtho-[1,2-c]-furan; 1,3,3a,4,5,6,7,7a-octahydroisobenzofuran; 3-methyl-2,2,3,5,5-pentacyanotetrahydrofuran; 4,4,6,6-tetracyano-3a,4,6,6a-tetrahydrofuro[3,4-b]furan; 6,9-dibromo-1,1,3,3-tetracyano1,3,3a,9b-tetrahydronaphtho-[1,2-c]furan;4-methyl-7-isopropyl-1,1,3,3-tetracyano-1,3,3a,7a-tetrahydroisobenzofuran; 1,1,3,3-tetracyano-3,3a,8,8a-tetrahydro-1H-indeno[1,2-c]furan; 1,1,3,3-tetracyanocycloocta[c]furan; 1,1,3,3-tetracyano-1,3,3a,11b-tetrahydrophenanthro-[9,19-c]furan; 1,1,3,3-tetracyanocyclobuta[c]furan; 4,7-dichloro-1,1,3,3-tetracyano-1,3,3a,7a-tetrahydroisobenzofuran; 1,1,3,3-tetracyano-6,9-diethyl-1,3,3a,9b-tetrahydronaphtho[1,2-c]furan. Tetrahedron, 1968, 24, 2551-66: this discloses adducts of tetracyanoethylene oxide and the following compounds:

prop-2-ene; benzene; naphthalene; phenanthrene; but-1-ene; cyclopentadiene; furan; thiophene; bicycloheptadiene; durene; cyclopentene; indene; bicycloheptene; bicyclooctadiene; cycloheptatriene; cyclooctatetraene. The reference concerns the synthesis of the adducts and no use is proposed for them.

J.O.C., 1969, 34, 2732: this describes the reaction of tetracyanoethylene oxide with methyl erucate

(methyl cis-13-docosenoate) and methyl brassidate (methyl trans-13-doccsenoate), giving 2,2,5,5-tetracyano-3-(11-carbomethoxyundecyl)-4-octyltetrahydrofurans. No utility is suggested for the tetrahydrofurans.

Org. Mag. Resonance, 1971, 3, 383-8: this concerns the NMR spectra of cycloaddition adducts between tetracyanoethylene oxide and various norbornadiene derivatives. No use for the adducts is mentioned.

DE-OLS-2342468 and US patent no. 3923841: 2,2,5,5-tetracyanotetrahydrofuran is discussed as a precursor in the preparation of tetrasodium or tetrapotassium tetrahydrofuran-2,2,5,5-tetracarboxylate and hydrates thereof, useful as sequestrants and detergency builders. No other use of 2,2,5,5-tetracyanotetrahydrofuran is mentioned.

Acta. Chem. Scand., B29, (1975), 441-50: this discloses the cycloaddition products of tetracyanoethylene oxide and thiophene, some substituted thiophenes, furan, selenophene, benzo[b]-thiophene and benzo[b]furan. The reference concerns the synthesis of the products and the reaction mechanism; no use for the products is proposed.

US patent no. 3923679: this discloses 3-methoxycarbonyl-and 3,4-di(methoxycarbonyl)-2,2,5,5-tetracyanotetrahydrofurans as precursors in the preparation of the corresponding 2,2,5,5-tetracarboxylatetetrahydrofuran detergents.

Thus, the disclosure of the journal articles mentioned above is confined to the synthesis of tetracyanofurans, to their spectroscopic characterisation, and to the reaction mechanisms by which they are formed. The patent specifications relate to the preparation of the compounds and to their use as intermediates in the preparation of further compounds, generally carboxylate-substituted furans used as solvents, plasticizers and detergents and in manufacture of films and fibres.

It has now surprisingly been discovered that certain tetracyanofuran compounds have fungicidal activity.

According to the present invention there is provided a method of combating a fungus at a locus, characterised by treating the locus with a fungicidally effective amount of a compound of general formula I

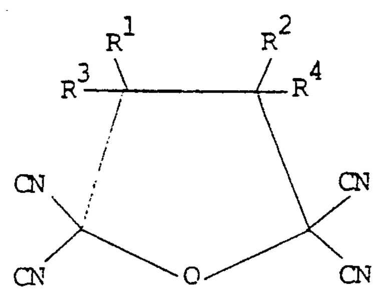

(I)

wherein each of $R^1$ and $R^2$ independently represents a hydrogen atom or an optionally substituted alkyl, alkenyl, alkynyl, aryl or aralkyl group; or an optionally substituted cycloalkyl, biocycloalkyl, cycloalkenyl or bicycloalkenyl group containing up to 12 ring atoms, in which at least one methylene group is optionally replaced by an oxygen atom or by a sulphone or sulphoxide group; or a group of general formula

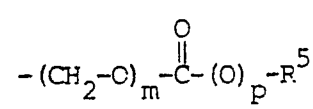

in which m is 0 and p is 0 or 1, or m is 1 and p is 0, and $R^5$ is an optionally substituted alkyl, alkenyl, aryl or aralkyl group; or $R^1$ and $R^2$ together with the interjacent carbon atoms form an optionally substituted cycloalkylene, bicycloalkylene, tricycloalkylene, cycloalkenylene, bicycloalkenylene or tricycloalkenylene group containing up to 18 ring atoms, in which at least one methylene group is optionally replaced by an oxygen or sulphur atom or by a sulphone or sulphoxide group; or an optionally substituted dihydronaphthylene, tetrahydronaphthylene, dihydrophenanthrylene or dihydroacenaphthenylene group; each of $R^3$ and $R^4$ independently represents a hydrogen atom or an alkyl group or together represent a single chemical bond; or $R^1$ and $R^3$ together with the interadjacent carbon atom form an optionally substituted cycloalkyl, bicycloalkyl, cycloalkenyl or bicycloalkenyl group containing up to 12 ring atoms, in which at least one methylene group is optionally replaced by an oxygen atom or a sulphone or sulphoxide group; provided that when $R^3$ and $R^4$ do not together represent a single chemical bond, at least one of $R^1$, $R^2$, $R^3$ and $R^4$ is a hydrogen atom.

In this specification, when the term optionally substituted is applied to an alkyl group substituents that

may be present include for example halogen atoms, and cyano and alkoxy groups, especially C(1-4) alkoxy.

In relation to cyclic groups, including aryl groups, substituents that may be present include for example halogen atoms, cyano, nitro and siloxy groups (for example, dimethyl-t-butyl-siloxy) and optionally substituted alkyl, alkoxy, alkoxycarbonyl, alkanoyl and alkanoyloxy groups especially those having 1 to 4 carbon atoms in the alkyl moiety. Preferred substituents include halogen atoms and trifluoromethyl, methoxy and methyl groups. An aryl group, where substituted, is preferably substituted by 1 to 3 substituent moieties.

The terms alkenylene, bicycloalkenylene and tricycloalkenylene are used in this specification to refer to bivalent groups having one or more, for example two or three, double bonds.

Specific examples of $R^1$ or $R^2$ being monocylic groups are cyclohexyl, 4-methyl-3-cyclohexen-1-yl and 2-furyl (falling within the definition above as a cycloalkenyl group having a methylene group replaced by an oxygen atom). An example of $R^1$ and $R^3$ together with the interjacent carbon atom being a bicyclic moiety is 3,3-dimethylbicyclo-[2.2.1]- hept-2-yl. Examples of $R^1$ and $R^2$ together with the interjacent carbon atoms being non-aromatic monocyclic, bicyclic or tricyclic groups are 1,2-cyclohexylene, 3,5-cyclohexadien-1,2-ylene, 4- and 5-methyl-3,5-cyclo- hexadien-1,2-ylene, 3,6-dimethyl-3,5-cyclohexadien-1,2-ylene, 4-ethoxycarbonyl-1,2-cyclohexylene, 4((4-nitrophenoxy)carbonyl)- 1,2-cyclohexylene, 4-acetyl-1,2-cyclohexylene, 4-([ethoxycarbony][4-chloro-2-nitrophenyl-acetamido]methyl)-1,2-cyclohexylene, 3-acetoxy-1,2-cyclohexylene, 1,2-cycloheptylene, bicyclo[2.2.1] hept-2,3-ylene, 5-acetoxy-bicyclo[2.2.1] hept-2,3-ylene, 1,2-cyclooctylene, 3-cycloocten-1,2-ylene, 5-cycloocten-1,2-ylene, 1,2-cyclododecylene, 4,5-(2-thioline)diyl and 8-spiro[(9,12-dioxolane)(bicyclo[3.2.1]oct-2,3-ylene)] groups. It will be apparent from the foregoing that bicyclic and tricyclic moieties may comprise fused rings, bridged rings or spiro unions.

Preferably each of $R^1$ and $R^2$ independently represents a hydrogen atom or an optionally substituted C-(1-16)alkyl, C(2-8)alkenyl or alkynyl, phenyl, naphthyl or benzyl group, or an optionally substituted cycloalkyl, cycloalkenyl, bicycloalkyl or bicycloalkenyl group containing up to 12 ring atoms, one of which may be an oxygen atom; or a group of general formula

$$-(CH_2-O)_m-\overset{\overset{O}{\|}}{C}-(O)_p-R^5$$

in which $R^5$ is an optionally substituted C(1-6) alkyl, phenyl or benzyl group; or $R^1$ and $R^2$ together with the interjacent carbon atoms form an optionally substituted cycloalkylene, cycloalkenylene, bicycloalkylene, bicycloalkenylene, tricycloalkylene or tricycloalkenylene group having up to 14 ring atoms, at least one of which may be a hetero atom selected from oxygen and sulphur; or an optionally substituted dihydronaphthylene, tetrahydronaphthylene, dihydrophenanthrylene or dihydroacenaphthenylene group; and each of $R^3$ and $R^4$ independently represents a hydrogen atom or a C(1-6) alkyl group or together represent a single chemical bond; or $R^1$ and $R^3$ together with the interjacent carbon atom represent a cycloalkyl, cycloalkenyl, bicycloalkyl or bicycloalkenyl group containing up to 12 ring atoms.

It is further preferred that each of $R^1$ and $R^2$ independently represents a hydrogen atom or an optionally substituted C(1-10) alkyl group, or a C(2-6) alkenyl group or an optionally substituted phenyl group, or a benzyl, benzoyl, furyl or naphthyl group; or an optionally substituted cyclohexyl group; or a group of formula $-CO_2R^6$ in which $R^6$ is an optionally substituted C(1-4) alkyl, phenyl or benzyl group; or $R^1$ and $R^2$ together with the interjacent carbon atoms form an optionally substituted cycloalkylene, bicycloalkylene or tricycloalkylene group containing up to 12 ring atoms or a cycloalkenylene group containing up to 8 ring atoms, wherein one or two of the ring atoms of each said group may be a hetero atom selected from oxygen or sulphur, or a dihydronaphthylene, tetrahydronaphthylene, dihydrophenanthrylene or dihydroacenaphthenylene group.

It is still further preferred that each of $R^1$ and $R^2$ independently represents a hydrogen atom, a C(1-4) alkyl group, an optionally substituted phenyl group, a benzyl group or a C(1-4) alkoxycarbonyl group; or $R^1$ and $R^2$ together with the adjacent carbon atoms form a cycloalkylene or bicycloalkylene group having up to 12 ring atoms, a cycloalkenylene group having up to 8 ring atoms, or a 9,10-dihydro-9,10-phenanthrylene or 1,2-dihydro-1,2-acenaphthenylene group.

Preferably, $R^3$ and $R^4$ together represent a single chemical bond, or one represents a hydrogen atom and the other represents a hydrogen atom or a C(1-4) alkyl group.

The compound of formula I may with particular advantage be a compound of the general formula

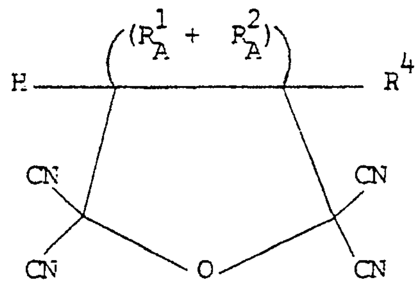

(II)

where $(R^1_A + R^2_A)$ together with the interjacent carbon atoms form an optionally substituted cycloalkylene, bicycloalkylene, tricycloalkylene, cycloalkenylene, bicycloalkenylene or tricycloalkenylene group containing up to 14 ring atoms, in which at least one methylene group is optionally replaced by an oxygen or sulphur atom or by a sulphone or sulphoxide group, or form an optionally substituted dihydronaphthylene, tetrahydronaphthylene, dihydrophenanthrylene or dihydroacenaphthenylene group; and $R^4$ is a hydrogen atom or a C(1-4) alkyl group. Preferably $R^4$ is a hydrogen atom or a methyl group, and $(R^1_A + R^2_A)$ together with the adjacent carbon atoms represent a 9,10-dihydro-9,10- phenanthrylene group, a 1,2-dihydro-1,2-acenaphthenylene group, or an optionally substituted cycloalkylene or bicycloalkylene group having up to 12 ring atoms, or an optionally substituted cycloalkenylene group having up to 8 ring atoms, especially a cyclohexadienylene group, optionally substituted by 1 to 4 moieties selected independently from halogen atoms, and alkyl and haloalkyl groups. Specific preferred compounds are 9,10-dihydro-9,10-phenanthrylene-2,2,5,5-tetracyanotetrahydrofuran, 1,2-dihydro-1,2-acenaphthenylene-2,2,5,5-tetracyanotetrahydrofuran, bicyclo[2.2.1]hept-2,3-ylene-2,2,5,5-tetracyanotetrahydrofuran and 2,5-dimethyl-2,4-cyclohexadien-1,2-ylene-2,2,5,5-tetracyanotetrahydrofuran.

The compound of formula I may advantageously be a compound of general formula

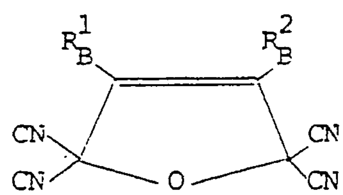

(III)

wherein each of $R^1_B$ and $R^2_B$ independently represents a hydrogen atom or an optionally substituted C(1-6) alkyl, phenyl, benzyl, naphthyl group, or a cycloalkyl group having up to 8 ring atoms, or a group of general formula

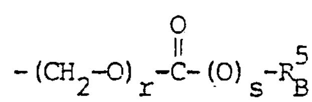

where $R^5_B$ is an optionally substituted C(1-6) alkyl or phenyl group, one of r and s is 0, and the other is 1. Especially preferred for $R^1_B$ and $R^2_B$ are hydrogen atoms and benzyl, phenyl and substituted phenyl groups, especially methyl-and halophenyl groups. In one preferred embodiment $R^1_B$ and $R^2_B$ are both benzyl groups.

Another subclass of compounds of general formula I is of the general formula

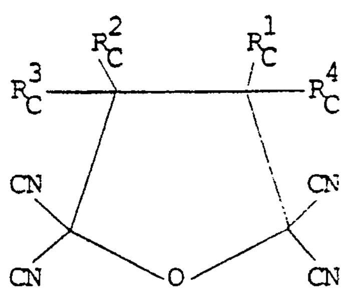

(IV)

wherein $R^1_C$ and $R^2_C$ are in the cis- or trans- configuration, preferably trans, and each independently

represents a hydrogen atom or an optionally substituted C(1-10) alkyl or alkenyl group, or an optionally substituted phenyl, benzyl or benzoyl group, a furyl group, or a group of formula

$$-(CH_2-O)_m-\overset{\overset{O}{\|}}{C}-(O)_p-R_C^5$$

where $R_C^5$ is an optionally substituted C(1-6) alkyl, phenyl or benzyl group, and m and p are as defined above; and each of $R_C^3$ and $R_C^4$ independently represents a hydrogen atom or a C(1-6) alkyl group; or $R_C^1$ and $R_C^3$ together with the interjacent carbon atom represent an optionally substituted cycloalkyl or bicycloalkyl moiety containing up to 10 ring atoms; provided that at least two of $R_C^1$ , $R_C^2$ , $R_C^3$ and $R_C^4$ represent hydrogen atoms.

Especially preferred groups for $R_C^1$ and $R_C^2$ are selected from phenyl, methylphenyl, methoxy- and dimethoxyphenyl, methoxycarbonyl and ethoxycarbonyl.

Where a cyclic moiety is referred to as containing up to a specified number of ring atoms, the minimum number of ring atoms the moiety can have is generally 4 for a monocyclic moiety and 5 for a bicyclic moiety, but is 5 if $R^1$ + $R^2$ together with the interadjacent carbon atoms is a cycloalkenylene group, and 7 if it is a bicycloalkenylene group, since such compounds according to the invention are made by the reaction of tetracyanoethylene oxide with cycloalkenes having at least two non-cumulated double bonds.

In the method according to the invention the locus preferably comprises plants subject to or subjected to fungal attack, seeds of such plants, or the medium in which the plants are growing or are to be grown. The plants are preferably vines.

The locus may conveniently be treated with the compound I at an application rate in the range 0.1-1kg/ha.

The invention also provides the use of a compound of formula I as defined above as a fungicide.

Further with accordance with the invention there is provided a fungicidal composition comprising a compound of formula I as defined above, in association with at least two carriers, at least one of which is a surface-active agent.

A composition according to the invention preferably contains from 0.5 to 95% by weight of active ingredient.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating fungicidal compositions may be used.

Suitable solid carriers include natural silicas such as diatomaceous earths; magnesium silicates, for example talcs; magnesium aluminum silicates, for example attapulgites and vermiculites; aluminium silicates, for example kaolinites, montmorillonites and micas; calcium carbonate; calcium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes, for example beeswax, paraffin wax, and chlorinated mineral waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Fungicidal compositions are often formulated and transported in a concentrated form which is subsequently diluted by the user before application. The presence of small amounts of a carrier which is a surface-active agent facilitates this process of dilution.

Of particular interest in enhancing the duration of the protectant activity of the compounds of this invention is the use of a carrier which will provide a slow release of the fungicidal compounds into the environment of the plant which is to be protected. Such slow-release formulations could, for example, be inserted in the soil adjacent to the roots of a vine plant, or could include an adhesive component enabling them to be applied directly to the stem of, for example, a vine plant.

A surface-active agent may be an emulsifying agent, a dispersing agent or a wetting agent; it may be nonionic or ionic. Examples of suitable surface-active agents include the sodium or calcium salts of

polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as sodium copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75%w of active ingredient and usually contain, in addition to solid inert carrier, 3-10%w of a dispersing agent and, where necessary, 0-10%w of stabiliser(s) and/or other additives such as penetrants or stickers. Dusts are usually formulated as a dust concentrate having a similar composition to that of a wettable powder but without a dispersant, and may be diluted in the field with further solid carrier to give a composition usually containing $\frac{1}{2}$-10%w of active ingredient. Granules are usually prepared to have a size between 10 and 100 BS mesh (1.676-0.152mm), and may be manufactured by agglomeration or impregnation techniques.

Generally, granules will contain $\frac{1}{2}$-25%w active ingredient and 0-10%w of additives such as stabilisers, slow release modifiers and binding agents. Emulsifiable concentrates usually contain, in addition to a solvent and, when necessary, co-solvent, 1-50%w/v active ingredient, 2-20%w/v emulsifiers and 0-20%w/v of other additives such as stabilisers, penetrants and corrosion inhibitors. Suspension concentrates are usually compounded so as to obtain a stable, non-sedimenting flowable product and usually contain 10-75%w of active ingredient, 0.5-15% of dispersing agent, 0.1-10%w of suspending agents such as protective colloids and thixotropic agents, 0-10%w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as antifreeze agents for water.

The compositions may also contain other ingredients, for example other compounds possessing pesticidal, especially insecticidal, acaricidal, herbicidal or fungicidal, properties.

Aqueous dispersions and emulsions, for example compositions obtained by diluting a wettable powder or a concentrate according to the invention with water, also lie within the scope of the present invention. The said emulsions may be of the water-in-oil or of the oil-in-water type, and may have a thick 'mayonnaise'-like consistency.

Known compounds of formula I may be prepared by the process employed in the journal references discussed earlier in this specification, for example, J.A.C.S., 1965, 87, 3657-65, or an analogous process.

Certain of the compounds of formula I are novel. Accordingly the invention also provides compounds of general formula V,

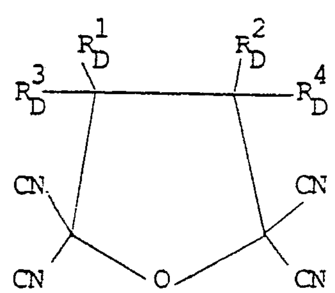

(V)

wherein $R_D^1$ is a substituted phenyl group and $R_D^2$ an alkoxycarbonyl group; or $R_D^1$ is a hydrogen atom and $R_D^2$ a fluorophenyl group; or each of $R_D^1$ and $R_D^2$ is independently selected from substituted phenyl groups, and optionally substituted benzyl groups; or $R_D^1$ and $R_D^2$ together with the adjacent carbon atoms represent a cycloalkylene group having 9 to 14 ring atoms or a cycloalkenylene group having from 8 to 12 ring atoms; and $R_D^3$ and $R_D^4$ both represent hydrogen atoms or together represent a single chemical bond.

Preferred novel compounds are

3,4-dibenzyl-2,2,5,5-tetracyano-2,5-dihydrofuran,

3,4-di(4-methylphenyl)-2,2,5,5-tetracyano-2,5-dihydrofuran,

2,2,4,4-tetracyano-3-oxabicyclo[10.3.0]pentadecane,
2,2,4,4-tetracyano-3-oxabicyclo[6.3.0]undec-6-ene and
2,2,4,4-tetracyano-3-oxabicyclo[6.3.0]undec-8-ene.

According to a further aspect of the invention there is provided a process for the preparation of a compound of general formula V as defined above, which comprises reacting tetracyanoethylene oxide with a compound of general formula VI

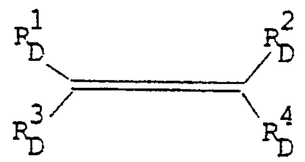

(VI)

where $R_D^1$, $R_D^2$, $R_D^3$ and $R_D^4$ are as defined above, at a temperature in the range 70°C to the reflux temperature. Preferably the reaction takes place in the presence of a solvent. The solvent used may be entirely inert to tetracyanoethylene oxide (for example 1,2-dichloroethane, 1,2-dibromoethane or 1-bromo-2-chloroethane) or may in principle be able to react with it, but will not do so because the compound (VI) is preferentially reactive with tetracyanoethylene oxide (for example benzene, toluene and xylene).

It is on occasion necessary for the reaction to take place under pressure, or in a sealed vessel, to ensure that it takes place at a suitable temperature, without loss of reagents through evaporation.

The invention will now be further illustrated by the following Examples:

Example 1
Preparation of 2,2,4,4-Tetracyano-3-oxabicyclo[10.3.0]pentadecane

A mixture of tetracyanoethylene oxide (0.72g, 5mmol) and cyclododecene (0.83g, 5mmol) in dry toluene (20ml) was refluxed under an atmosphere of nitrogen for 5 hours. The solvent was removed and the dark known residue was dissolved in hot ethanol and treated with charcoal. After filtration, the ethanolic solution was left aside for several days during which time 2,2,4,4 tetracyano-3-oxabicyclo[10.3.0]pentadecane separated as large clear crystals (0.5g), mp 105-6°C. Yield: 33%

| Analysis: | | | |
|---|---|---|---|
| Theory:<br>Found : | 69.7% C<br>69.3% C | 7.1% H<br>7.1% H | 18.1% N<br>17.8% N |

Example 2
Preparation of 3,4-Dibenzyl-2,2,5,5-tetracyano-2,5-dihydrofuran

A mixture of dibenzylacetylene (1.4g, 6.98mmol) and tetracyanoethylene oxide (1g, 6.94mmol) in 1,2-dibromoethane (16ml) was refluxed under a nitrogen atmosphere for 10 hours. After removing the solvent the residue was dissolved in dichloromethane (100ml) and treated with charcoal to give a yellow solid. Crystallization from ether-dichloromethane gave 3,4-dibenzyl-2,2,5,5-tetracyano-2,5-dihydrofuran as white crystals (0.75g), mp 156-7°C. Yield: 32%

| Analysis: | | | |
|---|---|---|---|
| Theory:<br>Found : | 75.4% C<br>75.5% C | 4.0% H<br>4.0% H | 16.0% N<br>15.8% N |

Example 3
Preparation of 2,2,4,4-Tetracyano-3-oxabicyclo[3.3.0]octane

Cyclopentene (0.5g, 7.3mmol) and tetracyanoethylene oxide (1.01g, 7mmol) were suspended in 1,2-dichloroethane (40ml) and heated at 150° in a sealed Pyrex (Trade Mark) tube for 17h. The solvent was removed and the residue was treated with boiling carbon tetrachloride and filtered. Upon concentration and cooling 2,2,4,4-tetracyano-3-oxabicyclo[3.3.0]octane crystallised as pale tan plates (0.7g), mp 105-7°C. Yield: 47%

| Analysis: | | | |
|---|---|---|---|
| Theory: | 62.3% C | 3.8% H | 26.4% N |
| Found: | 61.3% C | 3.5% H | 27.3% N |

Further compounds were prepared using the methods of Examples 1 to 3 described above. Information on these compounds is set out in the tables below. In these tables, the letter A denoted that a compound has been prepared by a method corresponding to that described in Example 1 above, that is, by refluxing in dry toluene under an atmosphere of nitrogen. The letter B denotes that a compound has been prepared by a method corresponding to that described in Example 2 above, that is, by relfuxing in dry 1,2-dibromoethane under an atmosphere of nitrogen. The letter C denotes that a compound has been prepared by a method corresponding to that described in Example 3 above, that is, by heating at 120-150°C in a sealed tube. The letter N denotes that a preparation has taken place without solvent. The tables state the unoptimised yields of the compounds, the purpose of the experiments being to produce compounds in quantities sufficient for fungicidal screening rather than to optimise reactions and extraction techniques to produce high percentage yields. The tables below also give the reaction time and the recrystallisation solvent used for each Example, using the following key:

a Benzene
b Diethylether
c Chloroform
d Carbon tetrachloride
e 1,2-Dichloroethane
f Cyclohexane
g Ethanol
h Diethylene-hexane
i Diethylether-dichloromethane
j Diethylether-chloroform
k Chloroform-carbon tetrachloride
l Column chromatography

TABLE I (A)

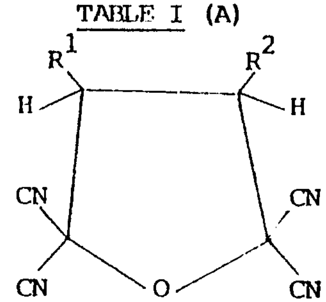

| Example No. | $(R^1 + R^2)$ ie$(R^1+R^2)$ together with the two interjacent carbon atoms | METHOD | TIME (hrs) | YIELD (%) | MPt. (°C) | SOL-VENT | ANALYSIS Calculated: Found: | C | H | N |
|---|---|---|---|---|---|---|---|---|---|---|
| 4 | 1,2-cyclohexylene | C | 16 | 70 | 115-116 | k | $C_{12}H_{10}N_4O$ | 63.5 | 4.4 | 24.7 |
| | | | | | | | | 63.6 | 4.3 | 24.9 |
| 5 | 1,2-cycloheptylene | A | 10 | 38 | 114-116 | d | $C_{13}H_{12}N_4O$ | 65.0 | 5.0 | 23.3 |
| | | | | | | | | 64.6 | 4.8 | 23.9 |
| 6 | 1,2-cyclooctylene | B | 6 | 50 | 112-113 | d | $C_{14}H_{14}N_4O$ | 66.1 | 5.5 | 22.0 |
| | | | | | | | | 66.1 | 5.5 | 21.8 |

Table 1 (continued)

| Example No. | 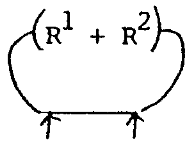 ie ($R^1$+$R^2$) together with the two interjacent carbon atoms | METHOD | TIME (hrs) | YIELD (%) | MPt. (°C) | SOLVENT | ANALYSIS Calculated: Found: | C | H | N |
|---|---|---|---|---|---|---|---|---|---|---|
| 7 | 3-cycloocten-1,2-ylene | B | 1 | 23 | 175 | k | $C_{14}H_{12}N_4O$ | 66.7 | 4.8 | 22.2 |
| | | | | | | | | 65.7 | 4.7 | 21.8 |
| 8 | 5-cycloocten-1,2-ylene | B | 2.5 | 34 | 136-7 | k | $C_{14}H_{12}N_4O$ | 66.7 | 4.8 | 22.2 |
| | | | | | | | | 66.2 | 4.6 | 21.9 |
| 9 | Bicyclo[2.2.1]hept-2,3-ylene | B | 7 | 53 | 195-8 (decomposed) | l | $C_{13}H_{10}N_4O$ | 65.5 | 4.2 | 23.5 |
| | | | | | | | | 65.2 | 4.9 | 23.1 |
| 10 | 5-Acetoxy-bicyclo[2.2.1]hept-2,3-ylene | B | 5 | 25 | 182-4 | k | $C_{15}H_{12}N_4O_3$ | 60.8 | 4.1 | 18.9 |
| | | | | | | | | 60.2 | 4.0 | 18.8 |
| 11 | 4,5-(2-thioline)diyl | C | 16 | 50 | 156-8 | e | $C_{10}H_4N_4OS$ | | | |
| 12 | 3,5-cyclohexadien-1,2-ylene | N | 96 | 10 | 160-2 | l | $C_{12}H_6N_4O$ | 64.9 | 2.7 | 25.2 |
| | | | | | | | | 64.8 | 2.5 | 24.6 |

Table 1 (continued)

| Example No. | 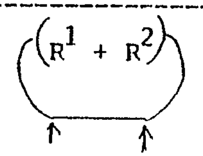 ie($R^1$+$R^2$) together with the two interjacent carbon atoms | METHOD | TIME (hrs) | YIELD (%) | MPt. (°C) | SOL-VENT | ANALYSIS Calculated: Found: | C | H | N |
|---|---|---|---|---|---|---|---|---|---|---|
| 13 | mixture of 4- and 5-methyl-3,-5-cyclohexadien-1,2-ylene | A | 23 | 61 | 129-132 (decomposed) | b | $C_{13}H_8N_4O$ | 66.1<br>65.9 | 3.1<br>3.2 | 23.7<br>24.1 |
| 14 | 1,2-dihydro-1,2-naphthylene | B | 4 | 80 | 170.5-173 | a | $C_{16}H_8N_4O$ | | | |
| 15 | 1,2,3,4-tetrahydro-1,2-naphthylene | | | | | | $C_{16}H_{10}N_4O$ | | | |
| 16 | 9,10-dihydro-9,10-phenanthrylene | B | 17 | 56 | 252-3 | a | $C_{20}H_{10}N_4O$ | 74.5<br>74.5 | 3.1<br>3.0 | 17.4<br>17.2 |
| 17 | 3-acetoxy-1,2-cyclohexylene | B | 6 | 25 | 146-7 | h | $C_{14}H_{12}N_4O_3$ | 59.1<br>59.2 | 4.2<br>4.0 | 19.7<br>19.3 |

Table 1 (continued)

| Example No. | 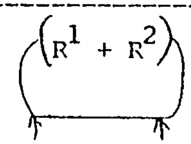 ie $(R^1+R^2)$ together with the two interjacent carbon atoms | METHOD | TIME (hrs) | YIELD (%) | MPt. (°C) | SOL-VENT | ANALYSIS Calculated: Found: | C | H | N |
|---|---|---|---|---|---|---|---|---|---|---|
| 18 | 4-ethoxycarbonyl-1,2-cyclohexylene | B | 5 | 67 | 112-6 | i | $C_{15}H_{14}N_4O_3$ | 60.4<br>60.5 | 4.7<br>4.7 | 18.8<br>18.9 |
| 19 | 4-((4-nitrophenoxy)carbonyl)-1,2-cyclohexylene | B | 6 | 35 | 170-5 | 1 | $C_{19}H_{13}N_5O_5$ | 58.3<br>56.4 | 3.3<br>3.8 | 17.9<br>16.8 |
| 20 | 4-([ethoxycarbonyl][4-chloro-2-nitrophenylacetamido]methyl)-1,2-cyclohexylene | B | 7 | 68 | 98-110 | 1 | $C_{23}H_{19}ClN_6O_6$ | 54.1<br>53.0 | 3.7<br>3.8 | 16.4<br>15.6 |
| 21 | 2,5-dimethyl-2,4-cyclohexadien-1,6-ylene | N | 20 | 45 | 218-9 | e | $C_{14}H_{10}N_4O$ | 67.2<br>67.2 | 4.0<br>3.9 | 22.4<br>22.4 |
| 22 | 4-acetyl-1,2-cyclohexylene | B | 6 | 16 | 175-6 | | $C_{14}H_{12}N_4O_2$ | 62.9<br>62.6 | 4.1<br>4.5 | 21.0<br>20.7 |

Table 1 (continued)

| Example No. | $(R^1 + R^2)$ ie $(R^1+R^2)$ together with the two interjacent carbon atoms | METHOD | TIME (hrs) | YIELD (%) | MPt. (°C) | SOL-VENT | ANALYSIS Calculated: Found: | C | H | N |
|---|---|---|---|---|---|---|---|---|---|---|
| 23 | 8-spiro[(9,12-dioxolane)-(bicyclo[3.2.1]oct-2,3--ylene)] | B | 4 | 56 | 200-2 | d | $C_{16}H_{14}N_4O_3$ | 61.9<br>61.4 | 4.5<br>4.2 | 18.1<br>18.1 |
| 24 | 1,2-dihydro-1,2-ace-naphthenlyene | B | 4 | 29 | 244-5 (decomposed) | | $C_{18}H_8N_4O$ | 73.0<br>73.1 | 2.7<br>2.7 | 18.9<br>18.9 |

Table 1 (continued)

| Example No. | $(R^1 + R^2)$ ie $(R^1+R^2)$ together with the two interjacent carbon atoms | METHOD | TIME (hrs) | MPt. (°C) | SOL-VENT | ANALYSIS Calculated: Found: | C | H | N |
|---|---|---|---|---|---|---|---|---|---|
| 81 | 2,5-diethyl-2,4-cyclohexadien-1,6-ylene | B | 17 | 172-3 | 1 | $C_{16}H_{14}N_4O$ | 69.1<br>68.9 | 5.0<br>5.3 | 20.1<br>20.4 |
| 82 | 2,5-diisopropyl-2,4-cyclohexadien-1,6-ylene | B | 20 | 205-7 | 1 | $C_{18}H_{18}N_4O$ | 70.6<br>70.7 | 5.9<br>6.0 | 18.3<br>18.3 |
| 83 | 2-methyl-5-bromo-2,4-cyclohexadien-1,6-ylene | B | 20 | 224-5 | 1 | $C_{13}H_7BrN_4O$ | 49.5<br>49.4 | 2.2<br>2.3 | 17.8<br>17.6 |
| 84 | 2,4,5-trimethyl-2,4-cyclohexadien-1,6-ylene | Neat | 12 | 160-2 | 1 | $C_{15}H_{12}N_4O$ | 68.2<br>67.8 | 4.5<br>4.6 | 21.2<br>21.0 |
| 85 | 2,4,5-triethyl-2,4-cyclohexadien-1,6-ylene | B | 5 | 98-100 | 1 | $C_{18}H_{18}N_4O$ | 70.6<br>70.2 | 5.9<br>5.9 | 18.3<br>18.2 |

TABLE I (B)

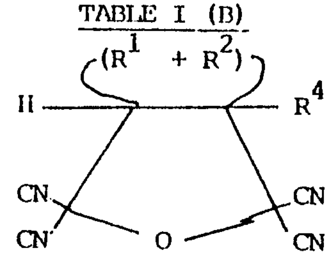

| Example No. | 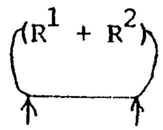 ie$(R^1+R^2)$ together with the two interjacent carbon atoms | $R^4$ | METHOD | TIME (hrs) | MPt. (°C) | SOL-VENT | ANALYSIS Calculated: Found: | C | H | N |
|---|---|---|---|---|---|---|---|---|---|---|
| 86 | 3-methyl-2,4-cyclohexadien-1,6-xylene | $CH_3$ | B | 20 | 98-100 | 1 | $C_{14}H_{10}N_4O$ | 67.2<br>68.0 | 4.0<br>4.3 | 22.4<br>21.0 |
| 87 | 3-ethyl-2,4-cyclohexadien-1,6-ylene | $C_2H_5$ | B | 17 | 96-98 | 1 | $C_{16}H_{14}N_4O$ | 69.1<br>69.9 | 5.0<br>5.1 | 20.1<br>20.3 |
| 88 | 2,3,5-trimethyl-2,4-cyclohexadien-1,6-xylene | $CH_3$ | Neat | 17 | 105-7 | 1 | $C_{16}H_{14}N_4O$ | 69.1<br>68.2 | 5.0<br>5.0 | 20.1<br>20.0 |

TABLE I (B) (continued)

| Example No. | ($R^1$ + $R^2$) ie($R^1$+$R^2$) together with the two interjacent carbon atoms | $R^4$ | METHOD | TIME (hrs) | MPt. (°C) | SOL-VENT | ANALYSIS Calculated: Found: | C | H | N |
|---|---|---|---|---|---|---|---|---|---|---|
| 89 | 2,4-dimethyl-2,4-cyclohexadien-1,6-ylene | $CH_3$ | Neat | 7 | 102-4 | 1 | $C_{15}H_{12}N_4O$ | 68.2<br>68.1 | 4.5<br>4.6 | 21.2<br>21.3 |
| 90 | 3-isopropyl-2,4-cyclohexadien-1,6-ylene | $C_3H_7i$ | B | 20 | 130-2 | 1 | $C_{18}H_{18}N_4O$ | 70.6<br>71.2 | 5.9<br>6.1 | 18.3<br>18.1 |
| 91 | 2,3,4,5-tetramethyl-2,4-cyclohexadien-1,6-ylene | $CH_3$ | Neat | 24 | 106-8 | 1 | $C_{12}H_{16}N_4O$ | 69.9<br>68.3 | 5.5<br>5.5 | 19.2<br>18.4 |

TABLE 2

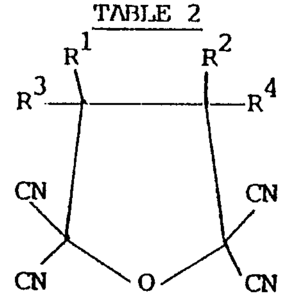

| Example No. | | METHOD | TIME (hrs) | YIELD (%) | MPt. (°C) | SOL-VENT | ANALYSIS Calculated: Found: | C | H | N |
|---|---|---|---|---|---|---|---|---|---|---|
| 25 | $R^1$ and $R^3$ together with the interjacent carbon atom form a 3,3-dimethyl-bicyclo-[2.2.1]hept-2-yl group; $R^2$, $R^4$: both hydrogen | B | 2.5 | 13 | 139-40 | 1 | $C_{16}H_{16}N_4O$ | 68.5<br>67.0 | 5.7<br>5.5 | 20.0<br>19.4 |
| 26 | $R^1$, $R^3$: both hydrogen<br>$R^2$: 4-methyl-3-cyclohexen-1-yl<br>$R^4$: methyl | B | 1.5 | 6.5 | 154-6 | 1 | $C_{16}H_{16}N_4O$ | 68.5<br>68.2 | 5.7<br>5.2 | 20.0<br>19.5 |

TABLE 3

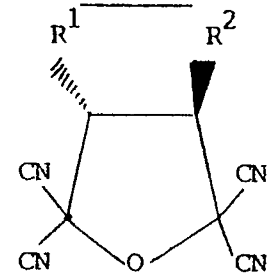

| Example No. | $R^1$ (trans unless stated) | $R^2$ | METHOD | TIME (hrs) | YIELD (%) | MPt. (°C) | SOLVENT | ANALYSIS Calculated: Found: | C | H | N |
|---|---|---|---|---|---|---|---|---|---|---|---|
| 27 | H | $CH_2CN$ | B | 17 | 33 | 151-2 | i | $C_{10}H_5N_5O$ | 56.9 | 2.4 | 33.2 |
| | | | | | | | | | 56.9 | 2.4 | 33.2 |
| 28 | H | $C_4H_9{}^n$ | A | 10 | 44 | oil | 1 | $C_{12}H_{12}N_4O$ | 63.1 | 5.3 | 24.6 |
| | | | | | | | | | 61.3 | 5.2 | 23.7 |
| 29 | H | $C_4H_9{}^t$ | A | 30 | 44 | oil | 1 | $C_{12}H_{12}N_4O$ | 63.1 | 5.3 | 24.55 |
| | | | | | | | | | 63.1 | 5.3 | 24.4 |
| 30 | H | $C_6H_{13}{}^n$ | A | 6 | 33 | oil | 1 | $C_{14}H_{16}N_4O$ | 65.6 | 6.2 | 21.9 |
| | | | | | | | | | 64.1 | 6.1 | 21.4 |
| 31 | H | $C_8H_{17}{}^n$ | A | 5 | 29 | oil | 1 | $C_{16}H_{20}N_4O$ | 67.6 | 7.0 | 19.7 |
| | | | | | | | | | 67.2 | 7.2 | 19.5 |

Table 3 (continued)

| Example No. | $R^1$ (trans unless stated) | $R^2$ | METHOD | TIME (hrs) | YIELD (%) | MPt. (°C) | SOL-VENT | ANALYSIS Calculated: Found: | C | H | N |
|---|---|---|---|---|---|---|---|---|---|---|---|
| 32 | H | $(CH_2)_2CH=CH_2$ | A | 18 | 50 | 64-5 | h | $C_{12}H_{10}N_4O$ | 63.7 | 4.4 | 24.8 |
| | | | | | | | | | 64.0 | 4.1 | 24.5 |
| 33 | H | phenyl | A | 6 | 27 | 142-4 | a | $C_{14}H_8N_4O$ | 67.7 | 3.2 | 22.6 |
| | | | | | | | | | 67.9 | 3.2 | 22.8 |
| 34 | acetoxy-methyl | acetoxymethyl [cis] | B | 6.5 | 56 | 104-6 | i | $C_{14}H_{12}N_4O_5$ | 53.2 | 3.8 | 17.7 |
| | | | | | | | | | 53.0 | 3.9 | 17.8 |
| 35 | phenyl | phenyl [cis] | A | 7 | 44 | 180-2 | d | $C_{20}H_{12}N_4O$ | 74.1 | 3.7 | 17.3 |
| | | | | | | | | | 72.6 | 3.1 | 18.0 |
| 36 | $C_2H_5$ | $C_2H_5$ | A | 5 | 38 | 104-5 | 1 | $C_{12}H_{12}N_4O$ | 63.2 | 5.3 | 24.6 |
| | | | | | | | | | 63.4 | 5.1 | 24.7 |
| 37 | $C_3H_7^n$ | $C_3H_7^n$ | A | 8 | 67 | 91-2 | 1 | $C_{14}H_{16}N_4O$ | 65.6 | 6.3 | 21.9 |
| | | | | | | | | | 65.8 | 6.6 | 21.9 |

Table 3 (continued)

| Example No. | $R^1$ (trans unless stated) | $R^2$ | METHOD | TIME (hrs) | YIELD (%) | MPt. (°C) | SOL-VENT | ANALYSIS Calculated: Found: | C | H | N |
|---|---|---|---|---|---|---|---|---|---|---|---|
| 38 | phenyl | phenyl | | | | | | | | | |
| 39 | 4-chloro-phenyl | 4-chlorophenyl | B | 5 | 58 | 242-3 | i | $C_{20}H_{10}Cl_2N_4O$ | 61.1<br>60.5 | 2.6<br>2.4 | 14.2<br>13.9 |

TABLE 4

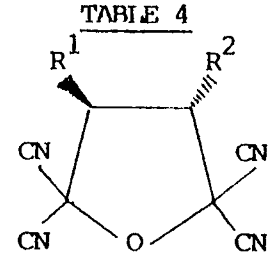

| Example No. | $R^1$ (trans unless stated) | $R^2$ | METHOD | TIME (hrs) | YIELD (%) | MPt. (°C) | SOL-VENT | ANALYSIS Calculated: Found: | C | H | N |
|---|---|---|---|---|---|---|---|---|---|---|---|
| 40 | $CH_3$ | $CO_2C_2H_5$ | B | 4.5 | 64 | 86-7 | j | $C_{12}H_{10}N_4O_3$ | 55.8 | 3.9 | 21.7 |
| | | | | | | | | | 55.6 | 3.5 | 21.5 |
| 41 | phenyl | benzoyl | B | 6.5 | 78 | 169-73 (decomposed) | c | $C_{21}H_{12}N_4O_2$ | 71.6 | 3.4 | 15.9 |
| | | | | | | | | | 71.6 | 3.1 | 15.8 |
| 42 | phenyl | $CH(OC_2H_5)_2$ | A | 25 | 57 | 100-1 | h | $C_{19}H_{18}N_4O_3$ | 65.1 | 5.2 | 16.0 |
| | | | | | | | | | 65.0 | 5.2 | 16.0 |
| 43 | phenyl | $CO_2CH_3$ | B | 5 | 58 | 131-3 | d | $C_{16}H_{10}N_4O_3$ | 62.7 | 3.3 | 18.3 |
| | | | | | | | | | 62.4 | 3.1 | 18.2 |
| 44 | phenyl | $CO_2C_2H_5$ | B | 3 | 78 | 104-5 | d | $C_{17}H_{12}N_4O_3$ | 63.7 | 3.8 | 17.5 |
| | | | | | | | | | 63.8 | 3.7 | 17.2 |

Table 4 (continued)

| Example No. | $R^1$ (trans unless stated) | $R^2$ | METHOD | TIME (hrs) | YIELD (%) | MPt. (°C) | SOL-VENT | ANALYSIS Calculated: Found: | C | H | N |
|---|---|---|---|---|---|---|---|---|---|---|---|
| 45 | phenyl | $CO_2CH_2C_6H_5$ | B | 4 | 65 | 107-8 | d | $C_{22}H_{14}N_4O_3$ | 69.1 | 3.7 | 14.7 |
| | | | | | | | | | 69.4 | 3.6 | 14.3 |
| 46 | 4-methyl-phenyl | $CO_2C_2H_5$ | B | 3.5 | 94 | 124-6 | d | $C_{18}H_{14}N_4O_3$ | 64.7 | 4.2 | 16.8 |
| | | | | | | | | | 64.8 | 3.9 | 16.8 |
| 47 | 3,4-dichloro-phenyl | $CO_2C_2H_5$ | A | 3.5 | 43 | 196-8 | h | $C_{17}H_{10}Cl_2N_4O_3$ | 52.4 | 2.6 | 14.4 |
| | | | | | | | | | 52.7 | 2.5 | 14.3 |
| 48 | 3-trifluoro-methylphenyl | $CO_2C_2H_5$ | B | 7 | 74 | 104-6 | b | $C_{18}H_{11}F_3N_4O_3$ | 55.7 | 2.9 | 14.4 |
| | | | | | | | | | 55.6 | 2.9 | 14.4 |
| 49 | 2-methoxy-phenyl | $CO_2C_2H_5$ | B | 2.5 | 47 | 88-9 | d | $C_{18}H_{14}N_4O_4$ | 61.7 | 4.0 | 16.0 |
| | | | | | | | | | 61.7 | 3.9 | 15.9 |
| 50 | 3-methoxy-phenyl | $CO_2C_2H_5$ | B | 4 | 40 | 69-70 | h | $C_{18}H_{14}N_4O_4$ | 61.7 | 4.0 | 16.0 |
| | | | | | | | | | 61.7 | 3.8 | 16.1 |
| 51 | 4-methoxy-phenyl | $CO_2C_2H_5$ | B | 6.5 | 91 | 94.6 | b | $C_{18}H_{14}N_4O_4$ | 61.7 | 4.0 | 16.0 |
| | | | | | | | | | 61.0 | 4.4 | 14.5 |

Table 4 (continued)

| Example No. | $R^1$ (trans unless stated) | $R^2$ | METHOD | TIME (hrs) | YIELD (%) | MPt. (°C) | SOLVENT | ANALYSIS Calculated: Found: | C | H | N |
|---|---|---|---|---|---|---|---|---|---|---|---|
| 52 | 3,4-dimethoxyphenyl | $CO_2C_2H_5$ | B | 4 | 42 | 134-6 | d | $C_{19}H_{16}N_4O_5$ | 60.0 | 4.2 | 14.7 |
| | | | | | | | | | 59.5 | 3.9 | 14.7 |
| 53 | 4-acetoxyphenyl | $CO_2C_2H_5$ | B | 6.5 | 41 | 135-7 | i | $C_{19}H_{14}N_4O_5$ | 60.3 | 3.7 | 14.8 |
| | | | | | | | | | 59.9 | 3.2 | 13.9 |
| 54 | 3-methoxyphenyl | $CO_2CH_2CCl_3$ | B | 6 | 41 | 133-4 | i | $C_{18}H_{11}Cl_3N_4O_4$ | 47.6 | 2.4 | 12.3 |
| | | | | | | | | | 45.7 | 2.3 | 11.4 |
| 55 | 2-furyl | 2,4,6-trichlorophenoxycarbonyl | B | 4.5 | 28 | 200-3 | i | $C_{19}H_7Cl_3N_4O_4$ | 49.4 | 1.5 | 12.1 |
| | | | | | | | | 49.3 | 1.6 | 12.3 | |
| 56 | $CO_2C_2H_5$ | $CO_2C_2H_5$ [cis] | B | 24 | 55 | 93-5 | d | $C_{14}H_{12}N_4O_3$ | 53.2 | 3.8 | 17.7 |
| | | | | | | | | | 53.2 | 3.7 | 17.5 |
| 57 | $CO_2C_2H_5$ | $CO_2C_2H_5$ | B | 24 | 70 | 96-8 | d | $C_{14}H_{12}N_4O_5$ | 53.2 | 3.8 | 17.7 |
| | | | | | | | | | 52.9 | 3.6 | 17.4 |
| 58 | 3-(dimethyl-t-butyl-siloxy)phenyl | $CO_2C_2H_5$ | B | 4 | 58 | 104.7 | h | $C_{23}H_{26}N_4O_4Si$ | 61.3 | 5.8 | 13.4 |
| | | | | | | | | | 61.1 | 5.7 | 13.7 |

TABLE 5

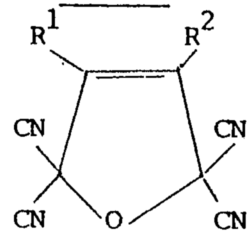

| Example No. | $R^1$ | $R^2$ | METHOD | TIME (hrs) | YIELD (%) | MPt. (°C) | SOL-VENT | ANALYSIS Calculated: Found: | C | H | N |
|---|---|---|---|---|---|---|---|---|---|---|---|
| 59 | H | phenyl | A | 5 | 41 | 129-31 | d | $C_{14}H_6N_4O$ | 68.3 | 2.4 | 22.8 |
| | | | | | | | | | 67.9 | 2.1 | 22.6 |
| 60 | $CH_3$ | phenyl | B | 16 | 58 | 103-5 | d | $C_{15}H_8N_4O$ | 69.2 | 3.1 | 21.5 |
| | | | | | | | | | 69.2 | 2.9 | 21.6 |
| 61 | H | 4-fluorophenyl | A | 4.5 | 50 | 182-4 | a | $C_{14}H_5FN_4O$ | 63.6 | 1.9 | 21.2 |
| | | | | | | | | | 63.8 | 2.0 | 21.5 |
| 62 | H | 4-methylphenyl | A | 4.5 | 12 | 114-5 | d | $C_{15}H_8N_4O$ | 69.2 | 3.1 | 21.5 |
| | | | | | | | | | 69.1 | 3.1 | 21.4 |
| 63 | H | (benzoyloxy)-methyl | B | 15 | 55 | 159-62 | c | $C_{16}H_8N_4O_3$ | 63.2 | 2.6 | 18.4 |
| | | | | | | | | | 63.0 | 2.5 | 18.3 |

Table 5 (continued)

| Example No. | $R^1$ | $R^2$ | METHOD | TIME (hrs) | YIELD (%) | MPt. (°C) | SOL-VENT | ANALYSIS Calculated: Found: | C | H | N |
|---|---|---|---|---|---|---|---|---|---|---|---|
| 64 | phenyl | phenyl | B | 17 | 67 | 179-82 | a | $C_{20}H_{10}N_4O$ | | | |
| 65 | phenyl | 3-methylphenyl | B | 17 | 22 | 128-30 | d | $C_{21}H_{12}N_4O$ | 75.0 | 3.6 | 16.7 |
| | | | | | | | | | 75.0 | 3.6 | 16.8 |
| 66 | phenyl | 2-(trifluoro-methyl)phenyl | B | 17 | 37 | 101-3 | d | $C_{21}H_9F_3N_4O$ | 64.6 | 2.3 | 14.4 |
| | | | | | | | | | 64.5 | 2.3 | 14.5 |
| 67 | phenyl | 2,5-dichloro-phenyl | B | 17 | 18 | 182-3 | d | $C_{20}H_8Cl_2N_4O$ | 61.4 | 2.0 | 14.3 |
| | | | | | | | | | 59.8 | 1.9 | 16.0 |
| 68 | phenyl | 4-methoxy-phenyl | B | 24 | 28 | 158-60 | 1 | $C_{21}H_{12}N_4O_2$ | 71.6 | 3.4 | 15.9 |
| | | | | | | | | | 71.6 | 3.4 | 15.7 |
| 69 | phenyl | 4-nitrophenyl | B | 17 | 50 | 167-8 | d | $C_{20}H_9N_5O_3$ | 65.4 | 2.5 | 19.1 |
| | | | | | | | | | 65.4 | 2.3 | 19.3 |
| 70 | 4-methyl-phenyl | 4-methylphenyl | B | 17 | 61 | 160-3 | 1 | $C_{22}H_{14}N_4O$ | 75.4 | 4.0 | 16.0 |
| | | | | | | | | | 75.9 | 4.1 | 16.2 |

Table 5 (continued)

| Example No. | $R^1$ | $R^2$ | METHOD | TIME (hrs) | YIELD (%) | MPt. (°C) | SOLVENT | ANALYSIS Calculated: Found: | C | H | N |
|---|---|---|---|---|---|---|---|---|---|---|---|
| 71 | 2-bromo-phenyl | 2-bromophenyl | B | 12 | 47 | 167-9 | i | $C_{20}H_8Br_2N_4O$ | 50.0 | 1.7 | 11.7 |
| | | | | | | | | | 49.9 | 1.7 | 11.6 |
| 72 | 2-naphthyl | 2-naphthyl | B | 24 | 12 | 162-4 | l | $C_{28}H_{14}N_4O$ | 79.6 | 3.3 | 13.3 |
| | | | | | | | | | 79.3 | 3.4 | 12.9 |
| 73 | cyclohexyl | cyclohexyl | B | 4 | 55 | 180-1 | d | $C_{20}H_{22}N_4O$ | 71.9 | 6.6 | 16.8 |
| | | | | | | | | | 71.2 | 6.6 | 17.2 |
| 74 | phenyl | $CO_2C_2H_5$ | B | 17 | 55 | 91-3 | d | $C_{17}H_{10}N_4O_3$ | 64.2 | 3.1 | 17.6 |
| | | | | | | | | | 64.0 | 3.0 | 17.8 |
| 75 | phenyl | 4-methoxyphen-oxycarbonyl | B | 17 | 20 | 115-6 | h | $C_{22}H_{18}N_4O_4$ | 66.7 | 3.0 | 14.1 |
| | | | | | | | | | 66.6 | 3.0 | 14.1 |
| 76 | phenyl | 4-nitrophenoxy-carbonyl | B | 17 | 35 | 140-2 | b | $C_{21}H_9N_5O_5$ | 61.3 | 2.2 | 17.0 |
| | | | | | | | | | 61.4 | 2.3 | 16.6 |
| 77 | phenyl | 3-trifluoro-methylphenoxy-carbonyl | B | 17 | 63 | 147-9 | | $C_{22}H_9F_3N_4O_3$ | 60.8 | 2.1 | 12.9 |
| | | | | | | | | | 60.6 | 2.2 | 13.0 |

Table 5 (continued)

| Example No. | $R^1$ | $R^2$ | METHOD | TIME (hrs) | YIELD (%) | MPt. (°C) | SOLVENT | ANALYSIS Calculated: Found: | C | H | N |
|---|---|---|---|---|---|---|---|---|---|---|---|
| 78 | phenyl | 2-chloro-4-trifluoromethylphenoxycarbonyl | B | 17 | 30 | 83-5 | h | $C_{22}H_8F_3ClN_4O_3$ | 56.4 | 1.7 | 12.0 |
| | | | | | | | | | 56.3 | 2.1 | 11.6 |
| 79 | 4-nitrophenyl | ethoxycarbonyl | B | 7 | 45 | 94-5 | f | $C_{17}H_9N_5O_5$ | 56.2 | 2.5 | 19.3 |
| | | | | | | | | | 55.5 | 2.2 | 21.8 |
| 80 | H | 4-pentynyl | B | 17 | 35 | 70-2 | h | $C_{13}H_8N_4O$ | 66.1 | 3.4 | 23.7 |
| | | | | | | | | | 66.1 | 3.5 | 23.1 |

Each of the compounds of Examples 1 to 91 above was prepared by a 1,3-dipolar addition reaction with tetracyanoethylene oxide. Tetracyanoethylene oxide may be prepared according to the method in J.A.C.S., 1965, 87, 3651. Many of the dipolarophile precursors were commercially available and the others were synthesised using known literature procedures. The compounds of formula I whose dipolarophile intermediates required to be synthesised are indicated below by reference to the Example number of the

resulting adduct.

Example 17

Dipolarophile known. Dict. Org. Compds.

Example 18

Dipolarophile known. Dict. Org. Compds.

Example 19
Preparation of 3-cyclohexenecarboxylic acid, 4-nitrophenyl ester

A solution of diisopropylcarbodiimide (1.7g, 5.5mmol) in dichloromethane (15ml) was added to a mixture of 3-cyclohexene carboxylic acid (1.5g, 11.9mmol) [Tetrahedron Lett., 1979, 399] and 4-nitrophenol (1.65g, 11.9mmol) in dichloromethane (45mm) cooled in ice. The resulting mixture was stirred at ambient temperature for 17 hours, glacial acetic acid (1ml) was added and the mixture was poured into water. The product was extracted into dichloromethane and the combined extracts were washed successively with 10% aqueous $NaHCO_3$, 3M HCl, water and brine. After drying over $MgSO_4$ one solvent was evaporated, ether was added and the mixture was filtered. Concentration of the filtrate and cooling afforded the ester as a white solid (2.15g) mp 62-6° C. Yield: 73%

| Analysis: | | | |
|---|---|---|---|
| Calculated: | 63.1% C | 5.3% H | 5.7% N |
| Found: | 62.6% C | 5.7% H | 6.6% N |

Example 20
Preparation of N-(4-Chloro-2-nitrophenylacetamido)-3-cyclohexene-1-glycine, ethyl ester

Thionyl chloride (4ml, 54.8mmol) was added to a suspension of 3-cyclohexene-1-glycine (3.75g, 24.2mmol)[JACS, 1958, 80, 2698] in ethanol (55ml) cooled in ice. The resulting mixture was refluxed for 21 hours, filtered and the solvent was removed under reduced pressure. The residue was dissolved in chloroform and the organic layer was washed successively with saturated sodium carbonate and brine. After drying over $MgSO_4$ and evaporation a viscous yellow oil remained which was purified by Kugelrohr distillation to give 3-cyclohexene-1-glycine, ethyl ester, as a colourless oil (3.5g, 79%) bp 190-210° C/2mmHg

| Analysis: | | | |
|---|---|---|---|
| Calculated: | 65.5% C | 9.3% H | 7.6% N |
| Found: | 63.4% C | 11.1% H | 7.5% N |

A solution of the ester (0.9g, 4.9mmol) in benzene (5ml) was added to a solution of 4-chloro-2-nitrobenzoyl chloride (1.1g, 5.0mmol) in benzene (20ml) containing triethylamine (1ml, 7.2mmol). The resulting mixture was refluxed for 1 hour, poured into water and the product extracted into ether. Usual work up gave a viscous orange oil which was purified by chromatography on silica eluting with dichloromethane: methanol (10:1 by volume) to give the amide as a mixture of diastereoisomers (1.5g) Yield: 83%

| Analysis: | | | |
|---|---|---|---|
| Theory: | 55.7% C | 5.2% H | 7.6% N |
| Found: | 54.8% C | 5.3% H | 7.0% N |

Example 34

Dipolarophile known. Angew. Chem. Int. Edn. Engl., 1978, 17, 569

Example 39

Dipolarophile known. Chem. Commun., 1976, 1053

Example 42

Dipolarophile known. Dict. Org. Compds.

Cinnamate intermediates used in the synthesis of many of the compounds of Table 3 may be prepared by either of two general methods. The first, for alkyl cinnamates, is to reflux the appropriate commercially available cinnamic acid with the appropriate alcohol in the presence of acid. The method is exemplified by the preparation of ethyl 3,4-dimethoxy cinnamate, used in the preparation of the compound of Example 52.

Preparation of ethyl 3,4-dimethoxy cinnamate

A solution of 3,4-dimethoxycinnamic acid (5g, 24mmol) and concentrated sulphuric acid (5ml) in ethanol (50ml) was refluxed for 17 hrs. The ethanol was removed under reduced pressure water (50ml) was added to the residue and the product was extracted into ether. The organic layer was washed successively with saturated aq. $NaHCO_3$, water and brine and dried over magnesium sulphate. Evaporation gave an oily residue which crystallised on cooling to give the ethyl ester as a fawn solid (5.26g), mp 149-152°C. Yield: 93%

| Analysis: | | |
|---|---|---|
| Calculated: | 66.1%C | 6.8%H |
| Found: | 66.3%C | 7.0%H |

The second general method, for aryl cinnamates, is to react the appropriate commercially available cinnamic acid with the appropriate aryl alcohol in the presence of dicyclohexyl carbodiimide and N,N-dimethylaminopyridine. This method is described in Angew. Chem. Int. Edn. Engl., 1978, 522.

Specific routes to cinnamate intermediates are also available. For example, ethyl 4-acetoxy cinnamate, used as an intermediate in the preparation of the compound of Example 53, was prepared as follows:

A solution of acetyl chloride (1ml, 14mmol) in dioxan (13ml) was added to a well-stirred mixture of ethyl 4-hydroxycinnamate (2g, 10.4mmol) and powdered sodium hydroxide (1g, 25mmol) in dioxan (25ml) to which had been added a few drops of "Aliquat 336" (Trade Mark), a phase transfer catalyst. The resulting mixture was stirred for 40 minutes, concentrated, poured into water (80ml) and the product extracted into ether. The combined extracts were washed with 3M hydrochloric acid, dried over magnesium sulphate and evaporated to give an oily residue. This was purified by chromatography on silica eluting with dichloromethane to give the diester as an oil which slowly crystallised(1.15g) mp 44-6°C. Yield: 47%

| Analysis: | | |
|---|---|---|
| Calculated: | 66.6% C | 6.0% H |
| Found: | 66.6% C | 5.9% H |

Example 61

Dipolarophile known. Can. J. Chem., 1963, 41, 1084

Example 62

Dipolarophile known. Can. J. Chem., 1963, 41, 1084

Example 63

Dipolarophile known. Ann. 1955, 596, 72

Example 64

Dipolarophile known. Tetrahedron, 1958, 3, 204

Example 65

Dipolarophile known. JACS., 1967, 89, 230

Example 69

Dipolarophile known. JACS., 1967, 89, 230

Example 70

Dipolarophile known. J. Prakt. Chem., 1911, 83, 215

Example 71

Dipolarophile known. J.A.C.S., 1959, 81, 3013

Example 72

Dipolarophile known. Bull. Chem. Soc., Jpn, 1970, 43, 3567

Example 73
Preparation of 1,2-Dicyclohexylethyne

A mixture of 1,2-dicyclohexylethanedione (12g. 53.8 mmol) and 85% hydrazine hydrate (7g) in propan-1-ol (50ml) was refluxed for 24 hours. Upon cooling the bis-hydrazone crystallised as a white solid (7.3g), mp 143-4° C. Yield: 54%

| Analysis: | | | |
|---|---|---|---|
| Calc:<br>Found: | 67.2% C<br>67.0% C | 10.4% H<br>10.5% H | 22.4% N<br>22.3% N |

The bis-hydrazone (5g, 20mmol) was suspended in benzene (50ml) and yellow mercuric oxide (8.7g) was added with rapid stirring. The resulting mixture was refluxed for 17 hours, allowed to cool and filtered. Concentration of the filtrate left an oily residue which was poured down a silica column eluting with hexane. 1,2-Dicyclohexylethyne was obtained as a colourless oil (2.8g). Yield: 74%

| Analysis: | | |
|---|---|---|
| Calc:<br>Found: | 88.4% C<br>88.2% C | 11.6% H<br>12.0% H |

Acetylinic esters used as dipolarophiles to prepare the compounds of Examples 74 to 79 may be prepared by standard esterification techniques from corresponding carboxylic acids and alcohols. By way of Example, the preparation of the compound of Example 78 was as follows:

Preparation of (2-chloro-4-trifluoromethylphenyl) phenylpropiolate

Diisopropylcarbodiimide (2.3g, 18.3 mmol) in $CH_2Cl_2$ (15ml) was added to a solution of phenylpropiolic acid (2.25g, 15.4mmol) and 2-chloro-4-trifluoromethylphenol (3.0g, 15.3mmol) in $CH_2Cl_2$ (30ml) cooled in ice. The ice-bath was removed after the addition, the reaction mixture was allowed to attain room temperature and was stirred for 3 hours. The mixture was filtered, ether was added to the filtrate and the

organic layer was washed successively with 3M HCl, 1M NaoH, water and brine. The organic layer was dried with $MgSO_4$ and evaporated to give a crude oil which was purified by flash chromatography eluting with hexane: ether (2:1 by volume) to give the title compound as a clear oil (4.2g, 84%)

ACTIVITY TESTS

The fungicidal activity of compounds of the invention was investigated by means of the following tests.

(a) Direct protectant activity against vine downy mildew (*Plasmopara viticola*; Pvp)

The test is a direct protectant one, using a foliar spray. The lower surfaces of leaves of whole vine plants (cv Cabernet Sauvignon) are sprayed with a solution of active material in 1:1v/v water/acetone containing 0.04%w "Triton X-155" (trade mark) (octylphenol polyoxyethylene surfactant), at a dosage of 1 kilogram of active material per hectare using a track sprayer which delivers 620 l/ha, and after a subsequent 24 hours under normal glasshouse conditions the lower surfaces of the leaves are inoculated by spraying with an aqueous solution containing $10^4$ zoosporangia/ml. The inoculated plants are kept for 24 hours in a high humidity compartment, 5 days under normal glasshouse conditions and then returned for a further 24 hours to high humidity. Assessment is based on the percentage of leaf area covered by sporulation compared with that on control leaves.

(b) Direct protectant activity against vine downy mildew persistence test. (*Plasmopara viticola*;P.v.per)

The test is the same as described under a) above with the difference that the inoculated plants are kept for 4 days in a high humidity compartment.

(c) Antisporulant activity against vine downy mildew (*Plasmopara viticola*; Pva)

The test is a direct antisporulant one using a foliar spray. The lower surfaces of leaves of whole vine plants (cv Cabernet Sauvignon) are inoculated by spraying with an aqueous suspension containing $10^4$ zoosporangia/ml 2 days prior to treatment with the test compound. The inoculated plants are kept for 24 hours in a high humidity compartment, and then 24 hours at glasshouse ambient temperature and humidity. When the plants are dry, infected leaves are sprayed on their lower surfaces with a solution of active material in 1:1 water/acetone containing 0.04%w/w "Triton X-155" (trade mark) (an octylphenol polyethoxylate surfactant). The spraying is carried out with a moving track sprayer which delivers 620 1/ha, and the concentration of active material is calculated to give an application rate of 1kg/ha. After spraying, the plants are returned to normal glasshouse conditions for 96 hours and are then transferred to the high humidity compartment for 24 hours to induce sporulation, prior to assessment. Assessment is visual and is based on the percentage of the leaf area covered by sporulation compared with that on control leaves.

(d) Direct protectant activity against vine grey mould (*Botrytis cinerea*; Bcp)

The test is a direct protectant one using a foliar spray and is effected as described under (a), with the difference that the leaves are inoculated by spraying with an aqueous solution containing $10^5$ conidia/ml.

(e) Activity against wheat leafspot (*Leptosphaeria nodorum*; Ln.)

The test is a direct antisporulant one, using a foliar spray. Leaves of wheat plants (cv Mardler), at the single leaf stage, are inoculated by spraying with an aqueous suspension containing $8 \times 10^5$ spores/ml. The inoculated plants are kept for 24 hours in a high humidity compartment prior to treatment. The plants are sprayed at a dosage of 1 kg. of active material per hectare using a track sprayer as described under (a). After drying, the plants are kept for 5 days under normal glasshouse conditions, followed by assessment. Assessment is based on the percentage of leaf area covered by sporulation compared with that on leaves of control plants.

(f) Activity against barley powdery mildew (*Erysiphe graminis f.sp. hordei*; Eg)

The test is a direct antisporulant one, using a foliar spray. Leaves of barley seedlings, cultivar Golden Promise, are inoculated by dusting with mildew conidia one day prior to treatment with the test compound. The inoculated plants are kept overnight at glasshouse ambient temperature and humidity prior to treatment. The plants are sprayed at a dosage of 1kg. of active material per hectare using a track sprayer as described under (a). After drying, plants are returned to a compartment at ambient temperature and humidity for up to 7 days, followed by assessment. Assessment is based on the percentage of leaf area covered by sporulation compared with that on leaves of control plants.

(g) Activity against apple powdery mildew (*Podosphaera leucotricha*; Pl)

The test is a direct anti-sporulant one using a foliar spray. The upper surfaces of leaves of whole apply seedlings are inoculated by spraying with an aqueous suspension containing $10^5$ conidia/ml 2 days prior to treatment with the test compound. The inoculated plants are immediately dried and kept at glasshouse ambient temperatures and humidity prior to treatment. The plants are sprayed at a dosage of

1 kilogram of active material per hectare using a track sprayer as described under (a). After drying the plants are returned to a compartment at ambient temperature and humidity for up to 9 days, followed by assessment. Assessment is based on the percentage of the leaf area covered by sporulation compared with that on leaves of control plants.

(h) Activity against rice leaf blast (Pyricularia oryzae Po)

The test is a direct eradicant one using a foliar spray. The leaves of rice seedlings (about 30 seedlings per pot) are sprayed with an aqueous suspension containing $10^5$ spores/ml 20-24 hours prior to treatment with the test compound. The inoculated plants are kept overnight in high humidity and then allowed to dry before spraying at a dosage of 1kg of active material per hectare using a track sprayer as described under (a). After treatment the plants are kept in a rice compartment at 25-30 °C and high humidity. Assessments are made 4-5 days after treatment and are based on the density of necrotic lesions and the degree of withering when compared with control plants.

(i) Activity against tomato early blight (Alternaria Solani As)

The test is a direct protectant one using a foliar spray. The upper surfaces of leaves of young tomato plants are sprayed with a solution of active material as described in (a) above. After 24 hours under normal glasshouse conditions, the upper surfaces of the leaves are inoculated by spraying with an aqueous suspension containing $10^4$ spores/ml. The inoculated plants are kept for 72 hours in a high humidity compartment are then removed in a high humidity compartment and are then removed to lower humidity (50-70% relative humidity). Assessment is made 8 days after inoculation.

(j) Activity against wheat eyespot (Pseudocereosporella herpotrichoides Ph)

The test is an in vitro one. Samples are prepared wherein 0.7 mls solution containing 2 mg active material dissolved in acetone is evenly dispersed in 20 ml molten half-strength potato dextrose agar (formed by dissolving 2g potato extract, 10g dextrose and 7.5g agar in 1 litre of water and sterilising for 15 minutes at 121 °C) and the resulting 20ml portions are allowed to set in 9cm petri dishes. The concentration of active material in the resulting samples is 100ppm. Upon setting, two plugs of 5mm diameter taken from the advancing edge of a stock plate of a 3 to 4 week old culture of P.herpotrichoides on full strength potato dextrose agar, incubated at 20-22 °C in darkness, are placed, equally spaced on the surface of each sample, mycelial side uppermost. The samples are incubated for 11 days at 20-22 °C in darkness before assessment. Diametric growth is measured with the width of the plug substracted and results compared with growth on a sample wherein 0.7ml acetone containing no active material is dispersed in 20ml half-strength potato agar.

(k) Activity against seedling wheat blight (Fusarium culmorium; FcS)

The test is an anti-sporulant one using a soil drench. Surface sterilised wheat seeds (var Waggoner) are inoculated by soaking in an aqueous suspension containing $7 \times 10^5$ spores/ml (60mg seed per 80ml suspension) at 22 °C for 6 hours. The seeds are then sown in pots (5 per pot) in sand at a depth of 1cm. 1 day after inoculation and planting the active material is applied at a rate of 10kg/ha by pouring on a soil drench (concentration 0.36g/1 active material in 12%v/v acetone/water) evenly over the sand. The pots are then transferred to glasshouse, kept at 25 °C and watered sparingly. 21 days after inoculation the resulting seedlings are removed from the pots and their roots are gently washed. Visual assessment is made based on lesion development on stem base and upper roots in comparison with control seedlings.

The extent of disease control in all the above tests is expressed as a rating compared with a diluent control according to the criteria:-

1 = about 50-80% disease control

2 = greater than 80% disease control

Results of the above tests are given in Table 6 following:

TABLE 6

| Ex. | Pvp | Pv.per | Pva | Bcp | Ln | Eg | Pl | Po | As | Ph | Fcs |
|---|---|---|---|---|---|---|---|---|---|---|---|
| 1 | 2 | 2 | | 2 | | | | | 2 | | |
| 2 | 2 | 2 | | | | | | | | | |
| 3 | 2 | 1 | | 2 | | | | | | | 2 |
| 4 | 2 | 2 | | | | | | | | | 1 |
| 5 | 1 | | | | | | | | | | |
| 6 | 2 | 2 | | 2 | | | 2 | | | | |
| 7 | | 2 | | 1 | | | | | 1 | | |
| 8 | | 2 | | 2 | 2 | 1 | | | | | |
| 9 | 2 | 2 | | 2 | | | | | | | |
| 10 | | 2 | | | 1 | | | | 1 | | |
| 11 | 2 | | 2 | | | | | | 2 | | 1 |
| 12 | 2 | | 1 | | | | | | | | |
| 13 | 1 | | | 2 | | | | | | | |
| 14 | 2 | | | 2 | | | | | | | |
| 15 | 2 | | | | | | | | | | |
| 16 | | 2 | | | | | | | | | |
| 17 | 2 | 1 | | | | | | | | | |
| 18 | 2 | 2 | | | | | | | | | |
| 19 | 2 | | | | | 1 | | | | | |
| 20 | 2 | | | 2 | | | 1 | | | | |
| 21 | 2 | 2 | | 2 | | | | | 2 | 2 | |
| 22 | | | | | | | | 1 | | 1 | |
| 23 | | | | 1 | | | | | | 1 | 2 |
| 24 | 2 | | | | | | | | 2 | 1 | |
| 25 | 2 | | | | | | | | 2 | | |
| 26 | 2 | | | | | | | | | | |
| 27 | | 2 | | | | | | | 1 | | |
| 28 | | | | | 2 | 1 | | | | | |
| 29 | | | | | | | | 2 | | | |

Table 6 (continued)

| Ex. | Pvp | Pv.per | Pva | Bcp | Ln | Eg | Pl | Po | As | Ph | Fcs |
|---|---|---|---|---|---|---|---|---|---|---|---|
| 30 | | | | | 1 | 2 | | | | | |
| 31 | | 1 | | | | 2 | | | | | 1 |
| 32 | 2 | | | | | 1 | | | | | |
| 33 | | | | 1 | | | | | | | 1 |
| 34 | 1 | 2 | | | | | | | | | |
| 35 | 2 | 2 | | | | | | | 2 | | |
| 36 | | | | 2 | | | | | | | |
| 37 | 2 | | | 1 | | | | | | | |
| 38 | 1 | | | | | | | | | | |
| 39 | | 2 | | | | | | | | | |
| 40 | | 1 | | | | | | | | | |
| 41 | | 1 | | | | | | 1 | | | |
| 42 | 2 | | | | | | | | | | |
| 43 | 1 | 2 | | 1 | | | | | | | |
| 44 | | | | | | | | | | | 2 |
| 45 | 1 | 2 | | | | | | | | | |
| 46 | | 1 | | | | | | | | | |
| 47 | | 2 | | | | | | | | | |
| 48 | | 1 | | | | | | | | | |
| 49 | 2 | 1 | | | | | | | | | |
| 50 | 2 | 2 | | | | | | | | | |
| 51 | 2 | | | | | | | | | | |
| 52 | 2 | 1 | | 1 | | | | | | | |
| 53 | 2 | | | | | | | | | | |
| 54 | 1 | | | | | | | | 1 | | |
| 55 | 2 | | | | | | | | | 2 | |
| 56 | | 1 | | | | | | | | | |
| 57 | 1 | | | 2 | | | | | | | |
| 58 | | | | | | 1 | | | | | |
| 59 | 2 | 2 | | 2 | | 1 | | | | | |

Table 6 (continued)

| Ex. | Pvp | Pv.per | Pva | Bcp | Ln | Eg | Pl | Po | As | Ph | Fcs |
|---|---|---|---|---|---|---|---|---|---|---|---|
| 60 | | | | 1 | | | | | | | |
| 61 | 2 | 2 | | | | | | | 1 | | |
| 62 | 2 | 2 | 2 | 2 | | 1 | | | | | |
| 63 | 2 | 1 | | 2 | | | | | | | 1 |
| 64 | 2 | 2 | | 1 | | | | | | | |
| 65 | 2 | 2 | | | | | | | | | |
| 66 | 2 | 2 | | | | 1 | | | | | |
| 67 | | | 1 | | | | | | | | |
| 68 | 2 | 2 | | | | | | | 2 | | |
| 69 | 1 | | | | | | | | | | 1 |
| 70 | | | | 1 | | | 1 | 2 | | | |
| 71 | 2 | | | | | | | | | | |
| 72 | | | | 1 | | | | | | | |
| 73 | | | | | | | | | 2 | | |
| 74 | | | | 2 | | | | | | | |
| 75 | | | | | | | | | | 1 | |
| 76 | | | | | | | | | | 1 | |
| 77 | | | | | | | | | 1 | | |
| 78 | | | | | | | | | | 2 | |
| 79 | 2 | 1 | | | | | | | | | |
| 80 | | | | | | 1 | | | | | |
| 81 | 2 | | | | | | | | 2 | | 1 |
| 82 | | | | | | | | | 1 | | |
| 83 | 1 | | 1 | | | | 2 | 2 | | 1 | |
| 84 | 2 | | | 2 | | | 1 | | 2 | 2 | |
| 85 | 1 | | | | | | 1 | | | 2 | 2 |
| 86 | 2 | | | 2 | | | 1 | | | 2 | 2 |
| 87 | 2 | | | | | | | | 2 | 2 | |
| 88 | 1 | | | | | | | | | | |

## Claims

1. A method of combating a fungus at a locus, characterised by treating the locus with a fungicidally effective amount of a compound of general formula I

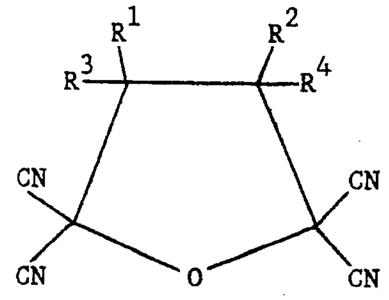

(I)

wherein each of $R^1$ and $R^2$ independently represents a hydrogen atom or an optionally substituted alkyl, alkenyl, alkynyl, aryl or aralkyl group; or an optionally substituted cycloalkyl, bicycloalkyl, cycloalkenyl or bicycloalkenyl group, each group containing up to 12 ring atoms, in which at least one methylene group is opticnally replaced by an oxygen atom or by a sulphone or sulphoxide group; or a group of general formula

$$-(CH_2-O)_m-\overset{\overset{O}{\|}}{C}-(O)_p-R^5$$

in which m is 0 and p is 0 or 1, or m is 1 and p is 0, and $R^5$ is an optionally substituted alkyl, alkenyl, aryl or aralkyl group; or $R^1$ and $R^2$ together with the interjacent carbon atoms form an optionally substituted cycloalkylene, bicycloalkylene, tricycloalkylene, cycloalkenylene, bicycloalkenylene, or tricycloalkenylene group, each group containing up to 18 ring atoms, in which at least one methylene group is optionally replaced by an oxygen or sulphur atom or by a sulphone or sulphoxide group, the terms cycloalkenylene, bicycloalkenylene and tricycloalkenylene being used to refer to bivalent groups having one or more double bonds; or an optionally substituted dihydronaphthylene, tetrahydronaphenthylene, dihydrophenanthrylene or dihydroacenaphthylene group; each of $R^3$ and $R^4$ independently represents a hydrogen atom or an alkyl group or together represent a single chemical bond; or $R^1$ and $R^3$ together with the interadjacent carbon atom form an optionally substituted cycloalkyl, bicycloalkyl, cycloalkenyl or bicycloalkenyl group, each group containing up to 12 ring atoms, in which at least one methylene group is optionally replaced by an oxygen atom or a sulphone or sulphoxide group; provided that when $R^3$ and $R^4$ do not together represent a single chemical bond, at least one of $R^1$, $R^2$, $R^3$ and $R^4$ is a hydrogen atom.

**2.** A method according to claim 1, wherein each of $R^1$ and $R^2$ independently represents a hydrogen atom or an optionally substituted C(1-16)alkyl, C(2-8)alkenyl or alkynyl, phenyl, naphthyl or benzyl group, or an optionally substituted cycloalkyl, cycloalkenyl, bicycloalkyl or bicycloalkenyl group containing up to 12 ring atoms, one of which may be an oxygen atom; or a group of general formula

$$-(CH_2-O)_m-\overset{\overset{O}{\|}}{C}-(O)_p-R^5$$

in which $R^5$ is an optionally substituted C(1-6) alkyl, phenyl or benzyl group; or $R^1$ and $R^2$ together with the interjacent carbon atoms form an optionally substituted cycloalkylene, cycloalkenylene, bicycloalkylene, bicycloalkenylene, tricycloalkylene or tricycloalkenylene group having up to 14 ring atoms, at least one of which may be a hetero atom selected from oxygen and sulphur; or an optionally substituted dihydronaphthylene, tetrahydronaphthylene, dihydrophenanthrylene or dihydroacenaphthenylene group; and each of $R^3$ and $R^4$independently represents a hydrogen atom or a C(1-6) alkyl group or together represent a single chemical bond; or $R^1$ and $R^3$ together with the interjacent carbon atom represent a cycloalkyl, cycloalkenyl, bicycloalkyl or bicycloalkenyl group containing up to 12 ring atoms.

**3.** A method according to claim 2 wherein each of $R^1$ and $R^2$ independently represents a hydrogen atom, a C(1-4) alkyl group, an optionally substituted phenyl group, a benzyl group or a C(1-4) alkoxycarbonyl

group; or $R^1$ and $R^2$ together with the adjacent carbon atoms form a cycloalkylene or bicycloalkylene group having up to 12 ring atoms, a cycloalkenylene group having up to 8 ring atoms, or a 9,10-dihydro-9,10-phenanthrylene or 1,2-dihydro-1,2-acenaphthenylene group; and $R^3$ and $R^4$ together represent a single chemical bond or one represents a hydrogen atom and the other represents a hydrogen atom or a C(1-4) alkyl group.

**4.** A method according to claim 3 wherein the compound has the general formula II:

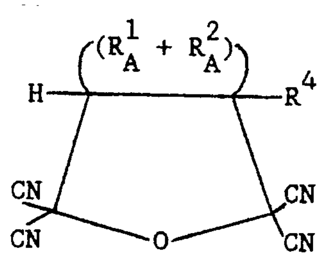

II

wherein ($R_A^1$ + $R_A^2$ ) together with the interjacent carbon atoms represent a 9,10-dihydro-9,10-phenanthrylene or 1,2-dihydro-1,2-acenaphthenylene group or a cyclohexadienylene group optionally substituted by 1 to 4 halogen atoms or C(1-4) alkyl or haloalkyl groups; and $R^4$ is a hydrogen atom or a methyl group.

**5.** A method according to any one of claims 1 to 4, in which the locus comprises plants subject to or subjected to fungal attack, seeds of such plants, or the medium in which the plants are growing or are to be grown, and is treated at an application rate in the range of 0.1 to 1 kg/ha.

**6.** The use of a compound of formula I as defined in any of claims 1 to 4, as a fungicide.

**7.** A fungicidal composition comprising an active ingredient and at least two carriers, at least one of which is a surface active agent, characterised in that the active ingredient is a compound of general formula I as defined in any one of claims 1 to 4.

**8.** A compound of the general formula V,

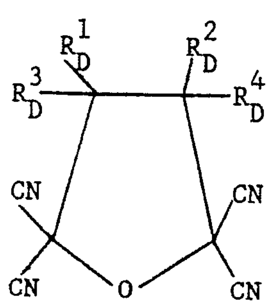

(V)

wherein $R_D^1$ is a substituted phenyl group and $R_D^2$ an alkoxycarbonyl group; or $R_D^1$ is a hydrogen atom and $R_D^2$ a fluorophenyl group; or each of $R_D^1$ and $R_D^2$ are independently selected from substituted phenyl groups, and optionally substituted benzyl groups; or $R_D^1$ and $R_D^2$ together with the adjacent carbon atoms represent a cycloalkylene group having from 9 to 14 ring atoms or a cycloalkenylene group having from 8 to 12 ring atoms, the term cycloalkenylene being used to refer to a bivalent group having one or more double bonds; and $R_D^3$ and $R_D^4$ both represent hydrogen atoms or together represent a single chemical bond.

**9.** A process for the preparation of a compound of formula V as claimed in claim 8, which comprises reacting tetracyanoethylene oxide with a compound of general formula VI

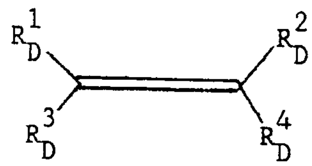

(VI)

where $R_D^1$, $R_D^2$, $R_D^3$ and $R_D^4$ are as defined in claim 8, at a temperature in the range 70°C to the reflux temperature.

**10.** A compound of the general formula V as claimed in claim 8, when produced by a process as claimed in claim 9.

**Revendications**

**1.** Une méthode de lutte contre un champignon en un lieu, caractérisée en ce qu'on traite le lieu avec une quantité efficace du point de vue fongicide d'un composé de formule générale I

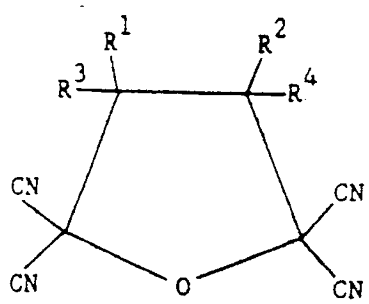

(I)

où chacun de $R^1$ et $R^2$ indépendamment représente un atome d'hydrogène ou un groupe alcoyle, alcényle, alcynyle, aryle ou aralcoyle éventuellement substitué ; ou un groupe cycloalcoyle, bicycloalcoyle, cycloalcényle ou bicycloalcényle éventuellement substitué, chaque groupe contenant jusqu'à 12 atomes dans le noyau, où au moins un groupe méthylène est éventuellement remplacé par un atome d'oxygène ou par un groupe sulfone ou sulfoxyde ; ou un groupe de formule générale

$$-(CH_2-O)_m-\overset{\displaystyle O}{\overset{\|}{C}}-(O)_p-R^5 .$$

où m est 0 et p est 0 ou 1, ou m est 1 et p est 0, et $R^5$ est un groupe alcoyle, alcényle, aryle ou aralcoyle éventuellement substitué ; ou $R^1$ et $R^2$ en même temps que les atomes de carbone situés entre eux forment un groupe cycloalcoylène, bicycloalcoylène, tricycloalcoylène, cycloalcénylène, bicycloalcénylène ou tricycloalcénylène éventuellement substitué, chaque groupe contenant jusqu'à 18 atomes dans le noyau, où au moins un groupe méthylène est éventuellement remplacé par un atome d'oxygène ou de soufre ou par un groupe sulfone ou sulfoxyde, les termes cycloalcénylène, bicycloalcénylène et tricycloalcénylène étant utilisés pour désigner des groupes bivalents ayant une ou plusieurs doubles liaisons ; ou un groupe dihydronaphtalène, tétrahydronaphtalène, dihydrophénanthrylène ou dihydroacénaphtylène ; chacun de $R^3$ et $R^4$ indépendamment représente un atome d'hydrogène ou un groupe alcoyle ou ils représentent ensemble une liaison chimique simple ; ou $R^1$ et $R^3$ en même temps que l'atome de carbone situé entre eux forment un groupe cycloalcoyle, bicycloalcoyle, cycloalcényle ou bicycloalcényle éventuellement substitué, chaque groupe contenant jusqu'à 12 atomes dans le noyau, où au moins un groupe méthylène est éventuellement remplacé par un atome d'oxygène ou un groupe sulfone ou sulfoxyde ; avec la condition que quand $R^3$ et $R^4$ ne représentent pas ensemble une liaison chimique simple, alors au moins un de $R^1$, $R^2$, $R^3$ et $R^4$ est un atome d'hydrogène.

2. Une méthode selon la revendication 1, dans laquelle chacun de $R^1$ et $R^2$ indépendamment représente un atome d'hydrogène ou un groupe C(1-16)alcoyle, C(2-8)alcényle ou alcynyle, phényle, naphtyle ou benzyle éventuellement substitué, ou un groupe groupe cycloalcoyle, cycloalcényle, bicycloalcoyle ou bicycloalcényle éventuellement substitué contenant jusqu'à 12 atomes dans le noyau, dont l'un peut être un atome d'oxygène ; ou un groupe de formule générale

$$-(CH_2-O)_m-\overset{O}{\overset{\|}{C}}-(O)_p-R^5$$

où $R^5$ est un groupe C(1-6)alcoyle, phényle ou benzyle éventuellement substitué ; ou $R^1$ et $R^2$ en même temps que les atomes de carbone situés entre eux forment un groupe cycloalcoylène, cycloalcénylène, bicycloalcoylène, bicycloalcénylène, tricycloalcoylène ou tricycloalcénylène ayant jusqu'à 14 atomes dans le noyau, dont l'un au moins peut être un hétéro-atome choisi parmi l'oxygène et le soufre ; ou un groupe dihydronaphtylène, tétrahydronaphtylène, dihydrophénanthrylène ou dihydroacénaphtylène éventuellement substitué ; et chacun de $R^3$ et $R^4$ indépendamment représente un atome d'hydrogène ou un groupe C(1-6)alcoyle ou ils représentent ensemble une liaison chimique simple ; ou $R^1$ et $R^3$ en même temps que l'atome de carbone situé entre eux représentent un groupe cycloalcoyle, cycloalcényle, bicycloalcoyle ou bicycloalcényle contenant jusqu'à 12 atomes dans le noyau.

3. Une méthode selon la revendication 2, dans laquelle chacun de $R^1$ et $R^2$ indépendamment représente un atome d'hydrogène, un groupe C(1-4)alcoyle, un groupe phényle éventuellement substitué, un groupe benzyle ou un groupe C(1-4)alcoxycarbonyle : ou $R^1$ et $R^2$ en même temps que les atomes de carbone adjacents forment un groupe cycloalcoylène ou bicycloalcoylène ayant jusqu'à 12 atomes dans le noyau, un groupe cycloalcénylène ayant jusqu'à 8 atomes dans le noyau ou un groupe 9,10-dihydro-9,10-phénanthrylène ou 1,2-dihydro-1,2-acénaphténylène : et $R^3$ et $R^4$ ensemble représentent une liaison chimique simple ou l'un représente un atome d'halogène et l'autre représente un atome d'hydrogène ou un groupe C(1-4)alcoyle.

4. Une méthode selon la revendication 3, dans laquelle le composé a la formule générale II :

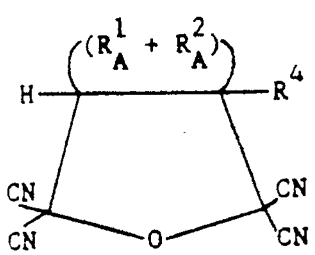

II

où ($R^1_A$ + $R^2_A$ ) en même temps que les atomes de carbone situés entre eux représentent un groupe 9,10-dihydro-9,10-phénanthrylène ou 1,2-dihydrol,2-acénaphténylène ou un groupe cyclohexadiénylène éventuellement substitué par 1 à 4 atomes d'halogènes ou groupes C(1-4)alcoyle ou haloalcoyle ; et $R^4$ est un atome d'hydrogène ou un groupe méthyle.

5. Une méthode selon l'une quelconque des revendications 1 à 4, dans laquelle le lieu comprend des plantes sensibles ou soumises à une attaque fongique, des semences de telles plantes, ou le milieu dans lequel les plantes poussent ou doivent pousser, et est traité à une dose d'application comprise entre 0,1 et 1,0 kg/ha.

6. L'utilisation d'un composé de formule I tel que défini dans l'une quelconque des revendications 1 à 4 comme fongicide.

7. Une composition fongicide comprenant un ingrédient actif et au moins deux véhicules, dont au moins un est un agent tensio-actif, caractérisée en ce que l'ingrédient actif est un composé de formule

générale I tel que défini dans l'une quelconque des revendications 1 à 4.

**8.** Un composé de la formule générale V

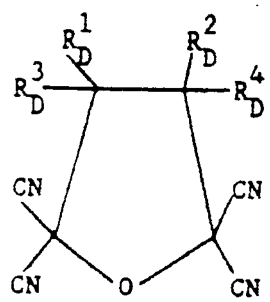

(V)

où $R_D^1$ est un groupe phényle substitué et $R_D^2$ un groupe alcoxycarbonyle ; ou $R_D^1$ est un atome d'hydrogène et $R_D^2$ un groupe fluorophényle ; ou chacun de $R_D^1$ et $R_D^2$ est choisi indépendamment parmi des groupes phényle substitués et des groupes benzyle éventuellement substitués ; ou $R_D^1$ et $R_D^2$ en même temps que les atomes de carbone adjacents représentent un groupe cycloalcoylène ayant de 9 à 14 atomes dans le noyau ou un groupe cycloalcénylène ayant de 8 à 12 atomes dans le noyau, le terme cycloalcénylène étant utilisé pour désigner un groupe bivalent ayant une ou plusieurs doubles liaisons ; et $R_D^3$ et $R_D^4$ représentent tous deux des atomes d'hydrogène ou représentent ensemble une liaison chimique simple.

**9.** Un procédé pour la préparation d'un composé de formule V tel que défini dans la revendication 8, qui comprend la réaction de l'oxyde de tétracyanoéthylène avec un composé de formule générale VI

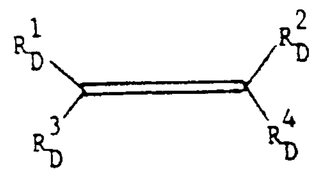

(VI)

où $R_D^1$ , $R_D^2$ , $R_D^3$ et $R_D^4$ sont tels que définis dans la revendication 8, à une température comprise entre 70 °C et la température de reflux.

**10.** Un composé de la formule générale V tel que défini dans la revendication 8 quand il est produit par un procédé selon la revendication 9.

## Patentansprüche

**1.** Verfahren zur Bekämpfung eines Pilzes an einem Ort, dadurch gekennzeichnet, daß der Ort mit einer fungizid wirksamen Menge einer Verbindung der allgemeinen Formel I

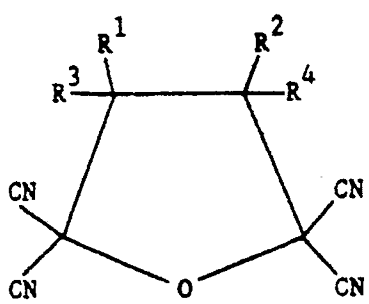

(I)

behandelt wird, in welcher Formel jeder der Reste $R^1$ und $R^2$ unabhängig voneinander ein Wasserstoff-

atom oder eine gegebenenfalls substituierte Alkyl-, Alkenyl-, Alkinyl-, Aryl- oder Aralkylgruppe; oder eine gegebenenfalls substituierte Cycloalkyl-, Bicycloalkyl-, Cycloalkenyl- oder Bicycloalkenylgruppe, wobei jeder Gruppe bis zu 12 Kohlenstoffatome enthält und worin wenigstens eine Methylengruppe gegebenenfalls durch ein Sauerstoffatom oder durch eine Sulfon- oder Sulfoxidgruppe ersetzt ist; oder eine Gruppe der allgemeinen Formel

$$-(CH_2-O)_m-\overset{\overset{\displaystyle O}{\|}}{C}-(O)_p-R^5$$

bedeutet, worin m den Wert 0 hat und p für 0 oder 1 steht oder m den Wert 1 hat und p für 0 steht und $R^5$ eine gegebenenfalls substituierte Alkyl-, Alkenyl-, Aryl- oder Aralkylgruppe darstellt; oder $R^1$ und $R^2$ zusammen mit den dazwischenliegenden Kohlenstoffatomen eine gegebenenfalls substituierte Cycloalkylen-, Bicycloalkylen-, Tricycloalkylen-, Cycloalkenylen-, Bicycloalkenylen- oder Tricycloalkenylengruppe mit jeweils bis zu 18 Ringatomen, worin wenigstens eine Methylengruppe gegebenenfalls durch ein Sauerstoff- oder Schwefelatom oder durch eine Sulfon- oder Sulfoxidgruppe substituiert ist, wobei die Ausdrücke Cycloalkenylen, Bicycloalkenylen und Tricycloalkenylen zweiwertige Gruppen mit einer oder mit mehreren Doppelbindungen bezeichnen; oder eine gegebenenfalls substituierte Dihydronaphthylen-, Tetrahydronaphthylen-, Dihydrophenanthrylen- oder Dihydroacenaphthylengruppe bedeuten; jeder der Reste $R^3$ und $R^4$ unabhängig voneinander ein Wasserstoffatom oder eine Alkylgruppe oder zusammen eine einfache chemische Bindung darstellen; oder $R^1$ und $R^3$ zusammen mit dem dazwischenliegenden Kohlenstoffatom eine gegebenenfalls substituierte Cycloalkyl-, Bicycloalkyl-, Cycloalkenyl- oder Bicycloalkenylgruppe mit jeweils bis zu 12 Ringatomen bedeuten, worin wenigstens eine Methylengruppe gegebenenfalls durch ein Sauerstoffatom oder eine Sulfon- oder Sulfoxidgruppe ersetzt ist; mit der Maßgabe, daß dann, wenn $R^3$ und $R^4$ nicht zusammen eine einfache chemische Bindung darstellen, wenigstens einer der Reste $R^1$, $R^2$, $R^3$ und $R^4$ ein Wasserstoffatom ist.

**2.** Verfahren nach Anspruch 1, worin jeder der Reste $R^1$ und $R^2$ unabhängig voneinander ein Wasserstoffatom oder eine gegebenenfalls substituierte C(1-16)Alkyl-, C(2-8)Alkenyl- oder -Alkinyl-, Phenyl-, Naphthyl- oder Benzylgruppe oder eine gegebenenfalls substituierte Cycloalkyl-, Cycloalkenyl-, Bicycloalkyl- oder Bicycloalkenylgruppe mit bis zu 12 Ringatomen bedeutet, von denen eines ein Sauerstoffatom sein kann; oder eine Gruppe der allgemeinen Formel

$$-(CH_2-O)_m-\overset{\overset{\displaystyle O}{\|}}{C}-(O)_p-R^5$$

bedeutet, worin $R^5$ eine gegebenenfalls substituierte C(1-6)Alkylgruppe, Phenylgruppe oder Benzylgruppe darstellt; oder $R^1$ und $R^2$ gemeinsam mit den dazwischenliegenden Kohlenstoffatomen eine gegebenenfalls substituierte Cycloalkylen-, Cycloalkenylen-, Bicycloalkylen-, Bicycloalkenylen-, Tricycloalkylen- oder Tricycloalkenylengruppe mit bis zu 14 Ringatomen ausbilden, von denen wenigstens eines ein unter Sauerstoff und Schwefel ausgewähltes Heteroatom sein kann; oder eine gegebenenfalls substituierte Dihydronaphthylen-, Tetrahydronaphthylen-, Dihydrophenanthrylen- oder Dihydroacenaphthenylengruppe bilden; und jeder der Reste $R^3$ und $R^4$ unabhängig voneinander für ein Wasserstoffatom oder eine C(1-6)Alkylgruppe steht oder gemeinsam eine einfache chemische Bindung darstellen; oder $R^1$ und $R^3$ zusammen mit dem dazwischenliegenden Kohlenstoffatom eine Cycloalkyl-, Cycloalkenyl-, Bicycloalkyl- oder Bicycloalkenylgruppe mit bis zu 12 Ringatomen bedeuten.

**3.** Verfahren nach Anspruch 2, worin jeder der Reste $R^1$ und $R^2$ unabhängig voneinander ein Wasserstoffatom, eine C(1-4)Alkylgruppe, eine gegebenenfalls substituierte Phenylgruppe, eine Benzylgruppe oder eine C(1-4)Alkoxycarbonylgruppe bedeutet; oder $R^1$ und $R^2$ gemeinsam mit den benachbarten Kohlenstoffatomen eine Cycloalkylen- oder Bicycloalkylengruppe mit bis zu 12 Ringatomen, eine Cycloalkenylengruppe mit bis zu 8 Ringatomen oder eine 9,10-Dihydro-9,10-phenanthrylen- oder 1,2-Dihydro-1,2-acenaphthenylengruppe bilden; und $R^3$ und $R^4$ zusammen eine einfache chemische Bindung darstellen oder ein Rest ein Wasserstoffatom und der andere Rest ein Wasserstoffatom oder eine C(1-4)-Alkylgruppe bedeuten.

**4.** Verfahren nach Anspruch 3, worin die Verbindung die allgemeine Formel II:

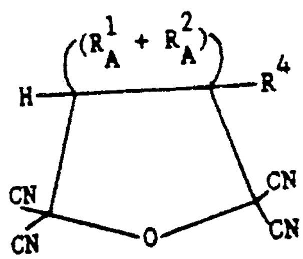

(II)

aufweist, worin ($R_A^1$ + $R_A^2$ ) gemeinsam mit den dazwischenliegenden Kohlenstoffatomen eine 9,10-Dihydro-9,10-phenanthrylen- oder 1,2-Dihydro-1,2-acenaphthenylengruppe oder eine gegebenenfalls durch 1 bis 4 Halogenatome oder C(1-4)Alkyl- oder -Halogenalkylgruppen substituierte Cyclohexadienylgruppe darstellt; und $R^4$ ein Wasserstoffatom oder eine Methylgruppe bedeutet.

**5.** Verfahren nach einem der Ansprüche 1 bis 4, worin der Ort Pflanzen umfaßt, die einen Pilzangriff unterliegen oder in Hinkunft unterliegen können, Samen solcher Pflanzen oder das Medium, worin die Pflanzen wachsen oder gezogen werden sollen, und mit einer Aufwandsmenge im Bereich von 0,1 bis 1 kg/ha behandelt wird.

**6.** Verwendung einer Verbindung der Formel I, wie in einem der Ansprüche 1 bis 4 definiert, als Fungizid.

**7.** Fungizide Zusammensetzung, umfassend einen wirksamen Bestandteil und wenigstens zwei Träger, von denen wenigstens einer ein oberflächenaktives Mittel ist, dadurch gekennzeichnet, daß der aktive Bestandteil eine Verbindung der allgemeinen Formel I, wie in einem der Ansprüche 1 bis 4 definiert, ist.

**8.** Verbindung der allgemeinen Formel V,

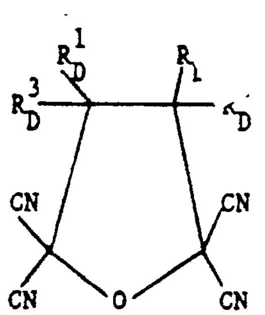

(V),

in welcher Formel $R_D^1$ eine substituierte Phenylgruppe und $R_D^2$ eine Alkoxycarbonylgruppe bedeuten; oder $R_D^1$ ein Wasserstoffatom- und $R_D^2$ eine Fluorphenylgruppe sind; oder jeder der Reste $R^1$ und $R^2$ unabhängig voneinander unter substituierten Phenylgruppen und gegebenenfalls substituierten Benzylgruppen ausgewählt ist; oder $R_D^1$ und $R_D^2$ zusammen mit den benachbarten Kohlenstoffatomen eine Cycloalkylengruppe mit 9 bis 14 Ringatomen oder eine Cycloalkenylengruppe mit 8 bis 12 Ringatomen bedeuten, wobei der Ausdruck Cycloalkenylen eine zweiwertige Gruppe mit einer oder mit mehreren Doppelbindungen bezeichnet; und $R_D^3$ und $R_D^4$ jeweils Wasserstoffatome oder gemeinsam eine einfache chemische Bindung darstellen.

**9.** Verfahren zur Herstellung einer Verbindung der Formel V, wie in Anspruch 8 beansprucht, welches ein Umsetzen von Tetracyanoethylenoxid mit einer Verbindung der allgemeinen Formel VI

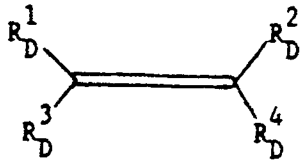

(VI),

worin $R_D^1$, $R_D^2$, $R_D^3$ und $R_D^4$ wie in Anspruch 8 definiert sind, bei einer Temperatur im Bereich von 70 °C bis zur Rückflußtemperatur umfaßt.

**10.** Verbindung der allgemeinen Formel V, wie in Anspruch 8 beansprucht, hergestellt nach einem Verfahren, wie in Anspruch 9 beansprucht.